(19) Europäisches Patentamt
European Patent Office
Office européen des brevets

(11) EP 2 932 946 A1

(12) EUROPEAN PATENT APPLICATION

(43) Date of publication:
21.10.2015 Bulletin 2015/43

(51) Int Cl.:
A61F 7/12 (2006.01)     A61M 5/44 (2006.01)

(21) Application number: 14001426.7

(22) Date of filing: 17.04.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: seiratherm GmbH
91074 Herzogenaurach (DE)

(72) Inventors:
• Roth, Mattias, Dr.
85376 Giggenhausen (DE)
• Reichthalhammer, Thomas, Dr.
85716 Unterschleissheim (DE)

(74) Representative: Stellbrink, Axel et al
Stellbrink IP
Ludwigstrasse 10
80539 München (DE)

(54) **Apparatus, system and method for controlling a temperature of a patient**

(57) Apparatus for adapting a body temperature, connected or connectable to an infusion device, preferably configured as an infusion needle, and a further temperature adaption device comprising a heatexchanger, preferably configured as an intravascular catheter or pad comprising the heatexchanger, wherein during use the infusion device and the further temperature adaption device share a fluid circuit portion through which fluid infusible to the patient and fluid intended for heat exchange in the further temperature adaption device flow; a controller adapted to control the body temperature by controlling the infusion of the infusion device and the heat exchange of the further temperature adaption device; and/or a fluid supply providing fluid for adapting the body temperature.

FIG. 1

**Description**

The disclosed technology relates to a device and a method for controlling or assisting to control the temperature of a patient.

Several technologies are known to control the temperature of a patient. For instance, it is known to control a temperature of a patient by heating or cooling the surface of a patient, for instance with disposable pads containing ice, water or gel. Other devices use temperature controlled pads, such as known pad systems.

Surface cooling based devices provide several advantages. The surface based cooling method is relatively simple to use. Ice pads might be used almost everywhere, for instance in an ambulance during medical primary care. Surface based treatment devices are comparatively inexpensive. Moreover, they are not invasive and easy to apply not involving time consuming steps during the set up. On the other hand, these methods/devices also incur certain drawbacks. For example, surface cooling only reaches comparably low cooling rates varying between 0,3 to 1,0°C/h for an average patient (cf. Kollmar et al., Therapeutische Hypothermie - Prinzip, Indikationen, praktische Anwendung, Unimed-Verlag 2011). Moreover, treating patients with ice pads in the long run is linked with higher labor costs and may result in skin irritations.

Using disposable ice pads also requires an increased logistic effort since these pre-cooled ice pads need to be provided/replaced on a regular basis during the treatment. In addition, the disposable ice pads require experienced clinical staff since the cooling rate of the disposable ice pads continuously changes during the treatment. This means that the clinical staff needs to monitor the actual body temperature of a patient on a regular basis and needs to adapt the ice pads accordingly. Due to relatively low cooling rates it is rather difficult to effectively control the rewarming of a patient at the end of a cooling treatment. This particularly applies to disposable ice pads which have a continuously changing cooling rate. Using surface based temperature control methods may further incur a considerably risk of overcooling the patient. This may result in serious complications, including arrhythmia, coagulopathy and an increased risk of infections. On the other hand, in order to avoid such overcooling, the clinical staff might not provide sufficient external cooling so that the patient does not reach the desired target temperature leading to inferior treatment results. In addition, known prior art temperature controlled surface temperature controlling devices generally tend to reduce the access to the patient for the clinical staff.

It is also known to control the temperature of a patient with intravascular catheters comprising a heat exchanger. Such catheters are generally controlled by controller using a temperature feedback from the patient.

Intravascular catheters are comparably expensive. They may require experienced clinical staff applying the invasive catheter into the patient's body. The setup of a catheter based temperature control device is relatively complex and time consuming. Such devices are generally not adapted to be used for medical primary care, for instance in an ambulance. Cooling rates of about 1,5°C/h might be obtained by an intravascular cooling catheter.

A third method of controlling the temperature of a patient is based on the infusion of a temperature controlled fluid. Document EP 2 203 136 A1 discloses an apparatus and a method for adjusting or stabilizing the temperature of a patient by infusion of a temperature controlled fluid such as a saline solution. Cooling a patient by infusing a fluid allows the highest cooling rates. For instance, a cooling rate up to 3,2°C/h may be obtained when infusing approximately 30 ml/kg. Thus, an average patient with a body weight of 70 kg would cool down from 37°C to 34°C within one hour by infusion of approximately 2,1 liter cold infusion fluid. The setup of the cooling device is relatively simple and quick. It can also be done in an ambulance for medical primary care. Compared to cooling a catheter applying infusion fluid is comparatively inexpensive and highly effective. Compared to other prior art technologies, infusion of temperature controlled fluids is the most appropriate technology to achieve high change rates of a patient temperature, particularly when compared to prior art intravascular cooling catheter.

While a certain minimal volume to be infused might be beneficial for certain patients, an excessive infused volume may negatively impact the patient's condition. The maximal volume of infusion fluid which can be infused into the patient is limited. The maximal volume of infusion fluid which may be infused into a patient depends on various physiological factors and varies from patient to patient. The maximal volume $V_{max}$ of infusion fluid (hereafter also referred to as maximal "amount" of infusion fluid) which may be provided to a patient per day may be influenced/limited by the renal function or by cardiac restrictions. The maximal amount of volume to be infused per day is generally set by the clinical staff after anamnesis. The maximal allowable volume to be infused might be, for instance, 5 to 6 liter infusion fluid per day. For patient with reduced renal function the maximal volume $V_{max}$ may be as low as 2 to 3 liters per day.

Document WO2006091284 A1 discloses a system for cooling a patient. The system comprises an intravenous heat exchange catheter, an external cooling pad and a bag and a pathway for infusing chilled saline. The saline is infused into the bloodstream of a patient while awaiting engagement of the catheter and/or pad with the patient. The immediate injection of saline ends when the heat exchange catheter or the external cooling pad is installed. The induced hypothermia subsequently is effected using the heat exchange catheter or the external cooling pad.

Document WO 02/098483 A1 discloses an intravascular catheter adapted to cool a patient.

The disclosed technology relates to control or assisting to control a temperature of a patient. This generally refers to heating and/or cooling (therapeutic or induced hypothermia) of a patient. Hereafter described is predominantly the scenario of actively cooling a patient. However, the technology equally applies to actively heating a patient.

There is a need, for different disease patters, to initially cool down a patient as fast as possible, for instance, to reduce or prevent neurologic damage by induced hypothermia as soon as possible. It is an object of the present invention to overcome or ameliorate the aforementioned drawbacks of the prior art and to provide an improved and/or alternative and/or additional method or device for controlling a temperature of a patient. It is one object of the present invention to provide an apparatus and a method for controlling a body temperature wherein the controlling accuracy of the body temperature is increased. In other words, it is desired to be able to respond quickly and reliably to deviations of the body temperature from the desired target temperature, for instance in the event of changing physiological conditions.

It is a further object of the present invention to provide a body temperature control apparatus and method wherein the volume to be infused is selectable by the clinician. A further preferred object is to provide a temperature control apparatus and method allowing minimizing the volume to be infused for a certain temperature treatment. It is a further object of the present invention to provide an apparatus and a system for controlling the body temperature at reduced cost. It is a further object to increase the ease of use and to reduce the setup time for the temperature control equipment

The object(s) underlying the present invention is(are) particularly solved by the features defined in the independent claims and aspects. The dependent claims and aspects relate to preferred embodiments of the present invention. Further additional and/or alternative aspects are discussed below.

1. Apparatus for controlling a body temperature ($T_b$) comprising a controller (100), the controller being adapted to control the body temperature ($T_b$) by controlling at least two different body temperature adaption devices (200, 300),

- wherein one body temperature adaption device is an infusion device (200); and
- wherein at least one further body temperature adaption device (300) is different from the one body temperature adaptation device and, preferably, not an infusion device.

The one body temperature adaptation device being an infusion device preferably encompasses blood/blood derivates and fluid infusion systems and/or preferably is a infusion system for infusing, e.g., saline or other balanced fluids like ringer's solution.

2. The apparatus of claim 1, wherein the infusion device (200) is adapted to add a volume of fluid to the patient's blood circuit, the volume of fluid having a different temperature than the body temperature ($T_b$).

3. The apparatus of claim 1 or 2, wherein the infusion device (200) is adapted to change the volume flow and/or the temperature of the fluid.

4. The apparatus of any one of the preceding claims, wherein the further body temperature adaption device (300) is a volume neutral body temperature adaption device.

5. The apparatus of any one of the preceding claims, wherein the further body temperature adaption device (300) is heat exchange based.

6. The apparatus of any one of the preceding claims, wherein the further body temperature adaption device (300) is at least one of: temperature adaption pad, cooling vests, head wraps, intravascular catheter, gas inhalation system, transnasal evaporative catheter systems, extra-corporal adaption of the blood temperature, blood warmer , temperature adaption mattress and/or blankets, heart-lung machine, temperature adaption tent, and peritoneal-lavage system.

7. The apparatus of any one of the preceding claims, wherein the controller (100) is adapted to control the body temperature ($T_b$) by simultaneously controlling the infusion device (200) and the at least one further body temperature adaption device (300).

8. The apparatus of any one of the preceding claims, wherein the controller (100) is adapted to selectively operate the infusion device (200) and the at least one further body temperature adaption device (300).

9. The apparatus of any one of the preceding claims, wherein the controller (100) is adapted to simultaneously operate the infusion device (200) and the at least one further body temperature adaption device (300).

10. The apparatus of any one of the preceding claims, wherein the controller (100) operates the infusion device (200) and/or the at least one further body temperature adaption device (300) to keep the actual body temperature $(T_{b\,act})$ close to a target body temperature $(T_{b\,tar})$.

11. The apparatus of any one of the preceding claims, wherein the controller is a closed loop controller adapted to compare the actual body temperature $(T_{b\,act})$ with a target body temperature $(T_{b\,tar})$.

12. The apparatus of any one of the preceding claims, wherein the controller (100) is adapted to operate the infusion device (200) and/or the further body temperature adaption device (300) under consideration of a minimal volume $(V_{min})$ and a maximal volume $(V_{max})$ to be applied to the patient during the target treatment time $(t_{treatment})$.

13. The apparatus of any one of the preceding claims, wherein the controller (100) is adapted to estimate an estimated volume $(V_{est})$ to be applied to the patient during the target treatment time $(t_{treatment})$.

14. The apparatus of claim 14, wherein the estimated volume $(V_{est})$ is estimated based on one or more, preferably all of the parameters: weight of the patient, temperature of the fluid to be infused, body-mass-index, target body temperature profile $(T_{b\,tar\,prof})$.

15. The apparatus of any one of the preceding claims, wherein the controller (100) is adapted to suggest and/or change an adaption measure in the event that the estimated volume $(V_{est})$ is not within the range defined by and including the minimal volume $(V_{min})$ and the maximal volume $(V_{max})$, the adaption measure causing a change in the estimated volume $(V_{est})$ relative to the minimal volume $(V_{min})$ and/or maximal volume $(V_{max})$.

16. The apparatus of claim 15, wherein said measure preferably results in a change of at least one of:

- range defined by and including the minimal volume $(V_{min})$ and the maximal volume $(V_{max})$,
- target body temperature profile $(T_{b\,tar\,prof\,new})$, including a change in length of the target temperature treatment period,
- corrected or new or estimated volume $(V_{est\,new})$,
- tolerance range for meeting the target body temperature,
- distribution of volume of fluid to be infused over time, particularly in correlation with the contribution to the change in temperature made by the further body temperature adaption device (300).

17. The apparatus of any one of the preceding claims, wherein the controller (100) is adapted to suggest and/or change to a shortened target treatment time $(t_{treatment\,new})$ in the event that the estimated volume $(V_{est})$ exceeds the maximal volume $(V_{max})$.

18. The apparatus of any one of the preceding claims, wherein the controller (100) is adapted to suggest and/or change to an extended target treatment time $(t_{treatment\,new})$ in the event that the estimated volume $(V_{est})$ is below the minimal volume $(V_{min})$.

19. The apparatus of any one of the preceding claims, wherein the controller (100) is adapted to suggest and/or change to a changed volume flow and/or a changed temperature of the infused fluid in the event that the estimated volume $(V_{est})$ is not within the range defined by and including the minimal volume $(V_{min})$ and the maximal volume $(V_{max})$.

20. The apparatus of any one of the preceding claims, wherein the controller (100) is adapted to increase the volume flow and to lower the temperature of the infused fluid so as to shift the estimated volume $(V_{est})$ above the minimal volume $(V_{min})$.

21. The apparatus of any one of the preceding claims, wherein the controller (100) is adapted to request the user to change the desired minimal volume $(V_{min})$ and/or the desired maximal volume $(V_{max})$ so that the estimated volume $(V_{est})$ is within the range defined by and including the minimal volume $(V_{min})$ and the maximal volume $(V_{max})$.

22. The apparatus of any one of the preceding claims, wherein the controller (100) is adapted to evaluate whether the actual body temperature $(T_{b\,act})$ is between

- a first lower threshold $(T_{l1})$ being lower than the target body temperature $(T_{b\,tar})$, and

- a first upper threshold ($T_{u1}$) being higher than the target body temperature ($T_{b\,tar}$).

23. The apparatus of claim 22, wherein the controller (100) is adapted to operate or to change the control parameters of the infusion device (200) and/or the further body temperature adaption device (300), if the actual temperature exceeds the first upper threshold ($T_{u1}$), to affect the actual body temperature ($T_{b\,act}$) to fall under the first upper threshold.

24. The apparatus of claim 22 or 23, wherein the controller (100) is adapted to operate or to change the control parameters of the infusion device (200) and/or the further body temperature adaption device (300), if the actual temperature falls below the first lower threshold ($T_{l1}$), to affect the actual body temperature ($T_{b\,act}$) to exceed the first lower threshold.

25. The apparatus of any one of the preceding claims, wherein the controller (100) is adapted to evaluate whether the actual body temperature ($T_{b\,act}$) is between

- a second lower threshold ($T_{l2}$) being lower than the first lower threshold, and
- a second upper threshold ($T_{u2}$) being higher than the first upper threshold.

26. The apparatus of claim 25, wherein the controller (100) is adapted to operate or to change the control parameters of the infusion device (200) and the further body temperature adaption device (300), if the actual body temperature ($T_{b\,act}$) falls below the second lower threshold, to affect the actual body temperature ($T_{b\,act}$) to exceed at least the second lower threshold.

27. The apparatus of claim 25 or 26, wherein the controller (100) is adapted to operate or to change the control parameters of the infusion device (200) and the further body temperature adaption device (300), if the actual body temperature ($T_{b\,act}$) exceeds the second upper threshold, to affect the actual body temperature ($T_{b\,act}$) to fall below at least the second upper threshold.

28. The apparatus of any one of the preceding claims, wherein the controller (100) is adapted to suggest and/or to change to different value(s) of the first and/or second lower threshold and/or of the first and/or second upper threshold, preferably in the event that the estimated volume ($V_{est}$) is not within the range defined by and including the minimal volume ($V_{min}$) and the maximal volume ($V_{max}$).
The same may apply for different estimated volumes for the plurality of phases each preferably having respective thresholds.

29. The apparatus of any one of the preceding claims, wherein the first lower threshold and the first upper threshold define a first target temperature tolerance range ($Ra_1$), and/or wherein the second lower threshold and the second upper threshold define a second target temperature tolerance range.

30. The apparatus of claim 29, wherein the controller (100) is adapted to suggest increasing or increases the first and/or second target temperature tolerance range, preferably in the event that the estimated volume ($V_{est}$) exceeds the maximal volume ($V_{max}$).

31. The apparatus of any one of the preceding claims, wherein the controller (100) is adapted to suggest decreasing or decreases the first and/or second target temperature tolerance range, preferably in the event that the estimated volume ($V_{est}$) exceeds the minimal volume ($V_{min}$).

32. The apparatus of any one of the preceding claims, wherein the controller (100) is adapted to estimate a control sensitivity ($S_{200}$, $S_{300}$) of the infusion device (200) and/or of the further body temperature adaption device (300).

33. The apparatus of claim 32, wherein the control sensitivity ($S_{200}$, $S_{300}$) of the infusion device (200) and/or of the further body temperature adaption device (300) is/are pre-set.

34. The apparatus of any one of the preceding claims, wherein the controller (100) is adapted to estimate at least one first estimated volume portion ($V_{est\,1}$) of fluid to be infused with a first control sensitivity ($S_{200-1}$) of the infusion device (200), and wherein the controller (100) is adapted to estimate at least one second estimated volume portion ($V_{est\,2}$) of fluid to be infused with a second control sensitivity ($S_{200-2}$) of the infusion device (200), the first control sensitivity ($S_{200-1}$) being higher than the second control sensitivity ($S_{200-2}$).

35. The apparatus of claim 34, wherein the sum of the first estimated volume portion ($V_{est\ 1}$) and the second estimated Volume portion ($V_{est\ 2}$) is the estimated volume ($V_{est}$).

36. The apparatus of claim 34 and 35, wherein the controller (100) is adapted to at least partially only operate or suggests at least partially only operating the further body temperature adaption device (300) during time period(s) the infusion device (200) would operate or is operating with the second control sensitivity ($S_{200-2}$).
E.g. the controller may operate at least partially only pad 300 during heating time periods of an induced cooling treatment.

37. The apparatus of claim 36, wherein the controller (100) suggest at least partially only operating and/or at least partially only operates the further body temperature adaption device (300) during time period(s) the infusion device (200) would operate or is operating with the second control sensitivity ($S_{200-2}$) in the event that the estimated volume ($V_{est}$) exceeds the maximal volume ($V_{max}$) such that the changed estimated volume ($V_{est\ new}$) is equal or below the maximal volume ($V_{max}$).

38. The apparatus of any one of claim 34 to 36, wherein the controller (100) is adapted to suggest operating and/or to operate at least the infusion device (200) during time period(s) the infusion device (200) would operate or is operating with the first control sensitivity ($S_{200-1}$).
E.g., the controller may suggest operating infusion device 200 during cooling periods of an induced cooling treatment.

39. The apparatus of claim 38, wherein the controller (100) suggests operating and/or operates at least the infusion device (200) during time period(s) the infusion device (200) would operate or is operating with the first control sensitivity ($S_{200-1}$) in the event that the estimated volume ($V_{est}$) is within the range defined by and including the first estimated volume portion (Vest 1) and the maximal volume ($V_{max}$).
E.g., the controller may suggest cooling or may cool with infusion fluid if sufficient fluid is to be infused while using pad 300 for heating of an induced cooling treatment.

40. The apparatus of claim 39, wherein the controller (100) suggests at least partially only operating the further body temperature adaption device (300) during time period(s) the infusion device (200) would operate or is operating with the first or second control sensitivity ($S_{200-1}$, $S_{200-2}$) in the event that the estimated volume ($V_{est}$) does not exceed the first estimated volume portion ($V_{est\ 1}$).
E.g., cooling with infusion fluid is preferred; however pad heating and cooling is required to keep the estimated volume $V_{est}$ below the maximal volume $V_{max}$.

41. The apparatus of any one of the preceding claims, wherein the controller (100) is adapted to at least operate the infusion device (200) if the actual body temperature ($T_{b\ act}$) is above the first upper threshold thereby cooling the patient.

42. The apparatus of any one of the preceding claims, wherein the controller (100) is adapted to at least operate the further adaption device (300) if the actual body temperature ($T_{b\ act}$) is below the first lower threshold thereby heating the patient.

43. The apparatus of any one of the preceding claims, wherein the controller (100) is adapted to estimate a volume of cooling fluid ($V_{est\ cool}$) to be infused required for cooling the patient during the treatment, and/or to estimate a volume of heating fluid ($V_{est\ heating}$) to be infused required for heating the patient during the treatment.

44. The apparatus of claim 43, wherein the volume of cooling fluid ($V_{est\ cool}$) and the volume of heating fluid ($V_{est\ heating}$) amount for the estimated volume ($V_{est}$).

45. The apparatus of claim 44, wherein the controller (100) suggests at least partially only operating and/or at least partially only operates the further body temperature adaption device (300) if the actual body temperature ($T_{b\ act}$) is below the first lower threshold, e.g. heating periods in the event that the estimated volume ($V_{est}$) exceeds the maximal volume ($V_{max}$) to thereby change the estimated volume ($V_{est}$) such that it is equal or below the maximal volume ($V_{max}$).
E.g., the controller may operate at least partially only pad 300 during heating time periods of an induced cooling treatment.

46. The apparatus of any one of claims 41 to 45, wherein the controller (100) suggests operating and/or operates at least the infusion device (200) if the actual body temperature ($T_{b\ act}$) is above the first upper threshold, e.g. cooling periods, in the event that the estimated volume ($V_{est}$) is within the range defined by and including the volume of cooling fluid ($V_{est\,cool}$) and the maximal volume ($V_{max}$).

E.g., the controller may suggest cooling or cools with infusion fluid if sufficient fluid is to be infused while using pad 300 for heating of an induced cooling treatment.

47. The apparatus of any one of claims 41 to 44, wherein the controller (100) suggests at least partially only operating or at least partially only operates the further body temperature adaption device (300) if the actual body temperature ($T_{b\,act}$) is not within the first target temperature tolerance range in the event that the estimated volume ($V_{est}$) does not exceed the first estimated volume portion ($V_{est\,1}$).

48. The apparatus of any one of the preceding claims, wherein the apparatus is adapted to divide the treatment into a plurality of treatment phases, the phases comprising at least one of

- a cooling phase (C) for cooling down the patient to a target treatment body temperature ($T_{b\,tar\,treat}$);
- a holding phase (H) during which the target treatment body temperature ($T_{b\,tar\,treat}$) is constant; and/or
- a warming phase (W) for re-warming the patient to a desired target body temperature $T_b$ tar end at the end of treatment.

49. The apparatus of claim 48, wherein the apparatus is adapted to derive for each phase (C, H, W) at least one of the following parameters:

- an estimated volume ($V_{est\,C,H,W}$) to be infused during the respective phase (C, H, W);
- a minimal volume ($V_{min\,C,H,W}$) to be infused during the respective phase (C, H, W);
- a maximal volume ($V_{max\,C,H,W}$) to be infused during the respective phase (C, H, W);
- a first lower threshold being lower than the target body temperature ($T_{btar}$) for the respective phase (C, H, W);
- a first upper threshold being higher than the target body temperature ($T_{btar}$) for the respective phase (C, H, W);
- a second lower threshold being lower than the first lower threshold for the respective phase (C, H, W);
- a second upper threshold being higher than the first upper threshold for the respective phase (C, H, W);
- first and/or second target temperature tolerance range(s) defined by lower and upper threshold(s) for the respective phase (C, H, W);
- a control sensitivity ($S_{200\,C,H,W}$, $S_{300\,C,H,W}$) of the infusion device (200) andor of the further body temperature adaption device (300) for the respective phase (C, H, W);
- a first estimated volume portion ($V_{est\,1}$) of fluid to be infused with a first control sensitivity ($S_{200-1\,C,H,W}$) of the infusion device (200) for the respective phase (C, H, W); and/or
- a second estimated volume portion ($V_{est\,2\,C,H,W}$) of fluid to be infused with a second control sensitivity ($S_{200-2\,C,H,W}$) of the infusion device (200) for the respective phase (C, H, W), the first control sensitivity ($S_{200-1\,C,H,W}$) being higher than the second control sensitivity ($S_{200-2\,C,H,W}$).

50. The apparatus of claim 49, wherein the minimal volume ($V_{min\,C,H,W}$) and/or the maximal volume ($V_{max\,C,H,W}$) to be infused of at least one of the phases (C, H, W) is pre-set by the user.

51. The apparatus of claim 49 or 50, wherein the controller is adapted to operate the infusion device (200) and/or the further body temperature adaption device (300) under consideration of a minimal volume ($V_{min\,C,H,W}$) and a maximal volume ($V_{max\,C,H,W}$) of at least one of the phases (C, H, W).

52. The apparatus according to any one of claims 49 to 51, wherein infusion in one of the phases (C, H, W) is prioritised over others.

53. The apparatus according to any one of claims 49 to 52, wherein the infusion device (200) at least operates during the cooling phase (C) for cooling down the patient, preferably together with the at least one further temperature adaption device (300).

54. The apparatus of any one of the preceding claims, wherein the controller (100) is adapted to estimate a temperature adaption rate ($R_{200}$, $R_{300}$) of the infusion device (200) and/or of the further body temperature adaption device (300).

55. The apparatus of any one of the preceding claims, wherein the controller (100) is adapted to estimate a temperature adaption rate for increasing the body temperature ($R_{200\,heat}$, $R_{300\,heat}$) of the infusion device (200) and/or of the further body temperature adaption device (300).

56. The apparatus of any one of the preceding claims, wherein the controller (100) is adapted to estimate a temperature adaption rate for lowering the body temperature ($R_{200\ cool}$, $R_{300\ cool}$) of the infusion device (200) and/or of the further body temperature adaption device (300).

57. The apparatus of any one of the preceding claims 54 to 56, wherein the controller (100) is adapted to control the infusion device (200) and/or the further body temperature adaption device (300) under consideration of the temperature adaption rate ($R_{200}$, $R_{300}$) of the infusion device (200) and/or of the further body temperature adaption device (300).

58. The apparatus of any one of the preceding claims 54 to 57, wherein the controller (100) is adapted to control the infusion device (200) and the further body temperature adaption device (300) under consideration of their respective temperature adaption rates for increasing the body temperature ($R_{200\ heat}$, $R_{300\ heat}$) and for lowering the body temperature ($R_{200\ cool}$, $R_{300\ cool}$).

59. The apparatus of any one of the preceding claims 54 to 58, wherein the controller (100) is adapted to compare the temperature adaption rates ($R_{200}$, $R_{300}$) of the infusion device (200) and the further body temperature adaption device (300), and wherein the controller (100) is adapted to selectively operate the one of the infusion device (200) and the further body temperature adaption device (300) having the higher temperature adaption rate ($R_{200}$, $R_{300}$), preferably under consideration of a minimal volume ($V_{min}$) and a maximal volume ($V_{max}$) to be applied to the patient during the target treatment time ($t_{treatment}$), most preferably so that the estimated volume (Vest) is within the range defined by and including the minimal volume (Vmin) and the maximal volume (Vmax)].

60. The apparatus of any one of the preceding claims, wherein the comparison between temperature adaption rates ($R_{200}$, $R_{300}$) of the infusion device (200) and the further body temperature adaption device (300) is carried out for increasing the body temperature ($R_{200\ heat}$, $R_{300\ heat}$) and for lowering the body temperature ($R_{200\ cool}$, $R_{300\ cool}$).

61. The apparatus of any one of the preceding claims, wherein the controller (100) is adapted to change or to suggest to change the temperature adaption rate ($R_{200}$, $R_{300}$) of the infusion device (200) and/or of the further body temperature adaption device (300), preferably under consideration of a minimal volume ($V_{min}$) and a maximal volume ($V_{max}$) to be applied to the patient during the target treatment time ($t_{treatment}$), most preferably so that the estimated volume (Vest) is within the range defined by and including the minimal volume ($V_{min}$) and the maximal volume ($V_{max}$).

62. The apparatus of any one of the preceding claims, wherein the controller (100) is adapted to prioritize the control of the infusion device (200) over the further body temperature adaption device (300) and/or of the further body temperature adaption device (300) over the infusion device (200).

63. The apparatus of any one of the preceding claims, wherein the controller (100) is adapted to be used in different treatment modes.

64. The apparatus of claim 63, wherein the controller is adapted to vary the control accuracy of the actual body temperature ($T_{b\ act}$) during the treatment time depending on the treatment mode.

65. The apparatus of claim 64, wherein the control accuracy is varied by varying the first and/or second target temperature tolerance range(s).

66. The apparatus of claim 64 or 65, wherein the control accuracy is varied by varying the sensitivity of the infusion device (200) and/or the further body temperature adaption device (300).

67. The apparatus of any one of the preceding claims 64 to 66, wherein the controller (100) in a modus of treatment with high control accuracy controls the body temperature by operating at least the infusion device (200), and wherein the controller (100) in a modus of low control accuracy controls the body temperature, preferably predominantly by operating the further body temperature adaption device (300), and wherein the control accuracy during the period of treatment with high control accuracy is higher than during the period of low control accuracy.

68. The apparatus of any one of the preceding claims, wherein the controller (100) is adapted to check prior to operation of at least one of the at least two different body temperature adaption device (200, 300), whether the

estimated volume ($V_{est}$) is within the range defined by and including the minimal volume ($V_{min}$) and the maximal volume ($V_{max}$).

69. The apparatus of any one of the preceding claims, wherein the controller (100) is adapted to estimate the estimated volume ($V_{est}$) during the operation of the apparatus.

70. The apparatus of any one of the preceding claims, wherein the controller (100) is adapted to change operation of at least one of the at least two different body temperature adaption device (200, 300), if the estimated volume ($V_{est}$) is not within the range defined by and including the minimal volume ($V_{min}$) and the maximal volume ($V_{max}$).

71. Apparatus for adapting a body temperature ($T_b$), preferably according to any one of the preceding claims, connected or connectable to an infusion device (200), preferably configured as an infusion needle, and a further temperature adaption device (300) comprising a heatexchanger (310), preferably configured as an intravascular catheter or pad comprising the heatexchanger (310),
wherein during use the infusion device (200) and the further temperature adaption device (300) share:

- a fluid circuit portion (700) through which fluid infusible to the patient and fluid intended for heat exchange in the further temperature adaption device (300) flow;
- a controller (100) adapted to control the body temperature ($T_b$) by controlling the infusion of the infusion device (200) and the heat exchange of the further temperature adaption device (300); and/or
- a fluid supply (400) providing fluid for adapting the body temperature;

72. The apparatus, preferably according to claim 71, wherein the infusion device (200) and the further temperature adaption device (300) share:

- a fluid temperature adaption device (500) adapted to change both the temperature of the fluid infusible to the patient and the fluid intended for heat exchange in the further temperature adaption device (300);
- a pump (600) adapted to pump at least one of fluid infusible to the patient and fluid intended for heat exchange in the further temperature adaption device (300); and/or
- a housing (800).

73. The apparatus of any one of the preceding claims, wherein the infusion device (200) is adapted to add a volume of fluid to the patient's blood circuit, the volume of fluid having a different temperature than the body temperature ($T_b$).

74. The apparatus according to any one of the preceding claims, wherein the fluid infusible to the patient and the fluid intended for heat exchange in the further temperature adaption device (300) are the same biocompatible fluid, preferably NaCl or other balanced fluids like ringer's solution.

75. The apparatus according to any one of the preceding claims, wherein the infusion device (200) and the intravascular catheter (300) in fluid connection with the apparatus are adapted to be applied to blood vessels at different locations.

76. The apparatus according to any one of the preceding claims, wherein the intravascular catheter (300) is applied into the central venous system, e.g. via femoral, subclavian or internal jugular insertion, and wherein the infusion device (200) is applied at a peripheral location, e.g. a venous access at the crook of the arm.

77. The apparatus according to any one of the preceding claims, wherein the apparatus (100) is adapted to selectively and/or simultaneously operate the infusion with the infusion device (200) and the heat exchange with the further temperature adaption device (300).

78. The apparatus of any one of the preceding claims, wherein the controller (100) is adapted to control the body temperature ($T_b$) by simultaneously controlling the infusion with the infusion device (200) and the heat exchange with the further temperature adaption device (300).

79. The apparatus of any one of the preceding claims, further comprising at least one measuring device (220, 320) adapted to measure the pressure and/or the temperature of the fluid.

80. The apparatus of claim 79, further comprising two measuring devices (220, 320), one (220) measuring the pressure and/or the temperature of the fluid infusible to the patient and the other (320) measuring the pressure and/or the temperature of the fluid intended for heat exchange in the intravascular catheter.

81a. The apparatus of any one of the preceding claims, further comprising a shared connector, wherein the shared connector is adapted to mate with a corresponding connector shared by the infusion means (200) and the further temperature adaption device (300).

81b. The apparatus of any one of the preceding claims, the temperature adaption device 500 being a heat exchanger and/or wherein the fluid temperature may be changed by mixing two fluids of different temperatures.

82. Apparatus for controlling a body temperature ($T_b$) and preferably according to any one of the preceding claims 71 to 81, fluidly connected or connectable to an infusion device (200) and an further temperature adaption device (300), wherein the infusion device (200) and the further temperature adaption device (300) share a fluid circuit portion (700), the fluid circuit portion (700) at least being located

- downstream of a fluid temperature adaption device (500) adapted to change both the temperature of the fluid infusible to the patient and the fluid intended for heat exchange in the further temperature adaption device (300), and
- upstream of a heat exchanger (310) located in the further temperature adaption device (300).

83. The apparatus according to claim 82, wherein an actuating device (900) is located in the shared fluid circuit portion (700), the actuating device (900) being adapted to split the flow of fluid entering the actuating device (900) into a flow of fluid infusible to the patient and a flow of fluid intended for heat exchange in the further temperature adaption device (300).

84. The apparatus according to claim 82 or 83, wherein the actuating device (900) is a switch or valve, preferably adapted to adjust the flow of fluid infusible to the patient and/or the flow of fluid intended for heat exchange based on a signal received from a control unit (100).

85. The apparatus of any one of the preceding claims 71 to 84, wherein the apparatus comprises a shared controller (100) the controller (100) providing a control signal at least to the fluid temperature adaption device (500) and the actuating device (900).

86. The apparatus of any one of the preceding claims 71 to 85, wherein shared fluid circuit portion (700) also encompasses a shared flow path (P1) in the fluid temperature adaption device (500).

87. The apparatus of any one of the preceding claims 71 to 86, wherein shared fluid circuit portion (700) also encompasses a shared flow path (P2) located upstream the fluid temperature adaption device (500) and downstream a shared fluid supply (400).

88. Apparatus for controlling a body temperature ($T_b$) and preferably according to any one of the preceding claims 71 to 85, connected or connectable to an infusion device (200) and a further temperature adaption device (300), wherein the infusion device (200) and the further temperature adaption device (300) share a fluid temperature adaption device (500), wherein a flow of fluid infusible to the patient and a flow of fluid intended for heat exchange in the further temperature adaption device (300) are two separate fluid flows (2700, 3700) flowing through the fluid temperature adaption device (500).

89. The apparatus of claim 88, wherein the fluid temperature adaption device (500) is configured to independently adapt the temperature of the two separate fluid flows.

90. The apparatus of claim 88 or 89, wherein the fluid temperature adaption device (500) is configured to adapt the temperatures of the two separate fluid flows (2700, 3700) based on at least one control signal from one controller (100).

91. The apparatus of claim 88, 89 or 90, wherein the fluid temperature adaption device (500) is configured to adapt the temperatures of the two separate fluid flows (2700, 3700) based on at least one control signal from a controller (100) separately controlling temperature of the two separate fluid flows (2700, 3700).

92. The apparatus of any one of the preceding claims 71 to 85 or 88 to 91, further comprising two separate fluid supplies (410, 420) each of which in fluid communication with one of the two separate fluid flows (2700, 3700).

93. The apparatus of claim 92, wherein the two separate fluid supplies (410, 420) are received in one container (430), the container preferably being thermally isolated.

94. A shared tubing comprising a shared connector, the connector being adapted to be connect to the shared connector of the apparatus of claim 81, wherein the shared tubing further comprises one flow path connected or connectable to the further temperature adaption device (300) and one flow path connected or connectable to the infusion device (200).

95. The shared tubing of claim 94, further comprising an isolation further thermally isolating the flow path of the infusion fluid from the flow path flowing from the catheter (300) back to the apparatus.

96. System for adapting a body temperature ($T_b$) comprising:

- the apparatus in accordance with any one of the preceding claims 71 to 95;
- an infusion device (200); and
- an intravascular catheter (300).

97. System of claim 96, further comprising the shared tubing of claim 94 or 95.

98. The system of claim 96 or 97, wherein the infusion device (200) is located in or at the intravascular catheter (300), preferably so that a heat exchange portion (310) of the catheter (300) and the infusion device (200) are jointly insertable into the patient through the same opening.

99. The system of claim of any one of the preceding claims 96 to 98, further comprising body temperature sensors.

100. The system of claim of any one of the preceding claims 96 to 98, further comprising a portable memory device.

101. Method for controlling a body temperature (Tb) comprising the step of controlling the body temperature (Tb) by controlling at least two different body temperature adaption devices (200, 300),

- wherein one body temperature adaption device is an infusion device (200); and
- wherein at least one further body temperature adaption device (300) is different from the one body temperature adaptation device and, preferably, not an infusion device.

The one body temperature adaptation device being an infusion device preferably encompasses blood/blood derivates and fluid infusion systems and/or preferably is a infusion system for infusing, e.g., saline or other balanced fluids like ringer's solution.

102. The method of claim 101, wherein the infusion device (200) is adding a volume of fluid to the patient's blood circuit, the volume of fluid having a different temperature than the body temperature (Tb).

103. The method of claim 101 or 102, wherein the infusion device (200) is changing the volume flow and/or the temperature of the fluid.

104. The method of any one of the preceding claims, wherein the further body temperature adaption device (300) is a volume neutral body temperature adaption device.

105. The method of any one of the preceding claims, wherein the further body temperature adaption device (300) is heat exchange based.

106. The method of any one of the preceding claims, wherein the further body temperature adaption device (300) is at least one of: temperature adaption pad, temperature adaption tent, intravascular catheter, gas inhalation, extra-corporal adaption of the blood temperature, temperature adaption mattress and/or blankets, heart-lung machine, and peritoneal-lavage, cooling vests, head wraps, intravascular catheter, transnasal evaporative catheter systems, blood warmer.

107. The method of any one of the preceding claims, further comprising the step of controlling the body temperature (Tb) by simultaneously controlling the infusion device (200) and the at least one further body temperature adaption device (300).

108. The method of any one of the preceding claims, further comprising the step of selectively operating the infusion device (200) and the at least one further body temperature adaption device (300).

109. The method of any one of the preceding claims, further comprising the step of simultaneously operating the infusion device (200) and the at least one further body temperature adaption device (300).

110. The method of any one of the preceding claims, further comprising the step of operating the infusion device (200) and/or the at least one further body temperature adaption device (300) to keep the actual body temperature (Tb act) close to a target body temperature (Tb tar).

111. The method of any one of the preceding claims, wherein the controller is a closed loop controller adapted to compare the actual body temperature (Tb act) with a target body temperature (Tb tar).

112. The method of any one of the preceding claims, further comprising the step of operating the infusion device (200) and/or the further body temperature adaption device (300) under consideration of a minimal volume (Vmin) and a maximal volume (Vmax) to be applied to the patient during the target treatment time (ttreatment).

113. The method of any one of the preceding claims, further comprising the step of estimating an estimated volume (Vest) to be applied to the patient during the target treatment time (ttreatment).

114. The method of claim 114, wherein the estimated volume (Vest) is estimated based on one or more, preferably all of the parameters: weight of the patient, temperature of the fluid to be infused, and body-mass-index, the list eventually also including target body temperature profile (Tb tar prof).

115. The method of any one of the preceding claims, further comprising the step of suggesting and/or changing an adaption measure in the event that the estimated volume (Vest) is not within the range defined by and including the minimal volume (Vmin) and the maximal volume (Vmax), the adaption measure causing a change in the estimated volume (Vest) relative to the minimal volume (Vmin) and/or maximal volume (Vmax).

116. The method of claim 115, wherein said measure preferably results in a change of at least one of:

- range defined by and including the minimal volume (Vmin) and the maximal volume (Vmax),
- target body temperature profile (Tb tar prof new), including a change in length of the target temperature treatment period,
- corrected or new or estimated volume (Vest new),
- tolerance range for meeting the target body temperature,
- distribution of volume of fluid to be infused over time in correlation with the contribution to the change in temperature made by the further body temperature adaption device (300).

117. The method of any one of the preceding claims, further comprising the step of suggesting and/or changing to a shortened target treatment time (ttreatment new) in the event that the estimated volume (Vest) exceeds the maximal volume (Vmax).

118. The method of any one of the preceding claims, further comprising the step of suggesting and/or changing to an extended target treatment time (ttreatment new) in the event that the estimated volume (Vest) is below the minimal volume (Vmin).

119. The method of any one of the preceding claims, further comprising the step of suggesting and/or changing to a changed volume flow and/or a changed temperature of the infused fluid in the event that the estimated volume (Vest) is not within the range defined by and including the minimal volume (Vmin) and the maximal volume (Vmax).

120. The method of any one of the preceding claims, further comprising the step of increasing the volume flow and lowering the temperature of the infused fluid so as to shift the estimated volume (Vest) above the minimal volume (Vmin).

121. The method of any one of the preceding claims, further comprising the step of requesting the user to change the desired minimal volume (Vmin) and/or the desired maximal volume (Vmax) so that the estimated volume (Vest) is within the range defined by and including the minimal volume (Vmin) and the maximal volume (Vmax).

122. The method of any one of the preceding claims, further comprising the step of evaluating whether the actual body temperature (Tb act) is between

- a first lower threshold (Tl1) being lower than the target body temperature (Tb tar), and
- a first upper threshold (Tu1) being higher than the target body temperature (Tb tar).

123. The method of claim 22, further comprising the step of operating or changing the control parameters of the infusion device (200) and/or the further body temperature adaption device (300), if the actual temperature exceeds the first upper threshold (Tu1), to affect the actual body temperature (Tb act) to fall under the first upper threshold.

124. The method of claim 22 or 23, further comprising the step of operating or changing the control parameters of the infusion device (200) and/or the further body temperature adaption device (300), if the actual temperature falls below the first lower threshold (Tl1), to affect the actual body temperature (Tb act) to exceed the first lower threshold.

125. The method of any one of the preceding claims, further comprising the step of evaluating whether the actual body temperature (Tb act) is between

- a second lower threshold (Tl2) being lower than the first lower threshold, and
- a second upper threshold (Tu2) being higher than the first upper threshold.

126. The method of claim 125, further comprising the step of operating or changing the control parameters of the infusion device (200) and the further body temperature adaption device (300), if the actual body temperature (Tb act) falls below the second lower threshold, to affect the actual body temperature (Tb act) to exceed at least the second lower threshold.

127. The method of claim 25 or 26, further comprising the step of operating or changing the control parameters of the infusion device (200) and the further body temperature adaption device (300), if the actual body temperature (Tb act) exceeds the second upper threshold, to affect the actual body temperature (Tb act) to fall below at least the second upper threshold.

128. The method of any one of the preceding claims, further comprising the step of suggesting and/or changing to different value(s) of the first and/or second lower threshold and/or of the first and/or second upper threshold, preferably in the event that the estimated volume (Vest) is not within the range defined by and including the minimal volume (Vmin) and the maximal volume (Vmax).
The same may apply for different estimated volumes for the plurality of phases each preferably having respective thresholds.

129. The method of any one of the preceding claims, wherein the first lower threshold and the first upper threshold define a first target temperature tolerance range (Ra1), and/or wherein the second lower threshold and the second upper threshold define a second target temperature tolerance range.

130. The method of claim 129, further comprising the step of suggesting increasing or increases the first and/or second target temperature tolerance range, preferably in the event that the estimated volume (Vest) exceeds the maximal volume (Vmax).

131. The method of any one of the preceding claims, further comprising the step of suggest decreasing or decreases the first and/or second target temperature tolerance range, preferably in the event that the estimated volume (Vest) exceeds the minimal volume (Vmin).

132. The method of any one of the preceding claims, further comprising the step of estimating control sensitivity (S200, S300) of the infusion device (200) and/or of the further body temperature adaption device (300).

133. The method of claim 132, wherein the control sensitivity (S200, S300) of the infusion device (200) and/or of the further body temperature adaption device (300) is/are pre-set.

134. The method of any one of the preceding claims, further comprising the step of estimating at least one first estimated volume portion (Vest 1) of fluid to be infused with a first control sensitivity (S200-1) of the infusion device (200), and further comprising the step of estimate at least one second estimated volume portion (Vest 2) of fluid to be infused with a second control sensitivity (S200-2) of the infusion device (200), the first control sensitivity (S200-1) being higher than the second control sensitivity (S200-2).

135. The method of claim 34, wherein the sum of the first estimated volume portion (Vest 1) and the second estimated Volume portion (Vest 2) is the estimated volume (Vest).

136. The method of claim 134 and 135, further comprising the step of at least partially only operating or suggesting at least partially only operating the further body temperature adaption device (300) during time period(s) the infusion device (200) would operate or is operating with the second control sensitivity (S200-2).
E.g. the controller may operate at least partially only pad 300 during heating time periods of an induced cooling treatment.

137. The method of claim 36, further comprising the step of suggesting at least partially only operating and/or at least partially only operates the further body temperature adaption device (300) during time period(s) the infusion device (200) would operate or is operating with the second control sensitivity (S200-2) in the event that the estimated volume (Vest) exceeds the maximal volume (Vmax) such that the changed estimated volume (Vest new) is equal or below the maximal volume (Vmax).

138. The method of any one of claim 34 to 36, further comprising the step of suggesting operating and/or operating at least the infusion device (200) during time period(s) the infusion device (200) would operate or is operating with the first control sensitivity (S200-1).
E.g., the controller may suggest operating infusion device 200 during cooling periods of an induced cooling treatment.

139. The method of claim 138, further comprising the step of suggesting operating and/or operates at least the infusion device (200) during time period(s) the infusion device (200) would operate or is operating with the first control sensitivity (S200-1) in the event that the estimated volume (Vest) is within the range defined by and including the first estimated volume portion (Vest 1) and the maximal volume (Vmax).
E.g., the controller may suggest cooling or may cool with infusion fluid if sufficient fluid is to be infused while using pad 300 for heating of an induced cooling treatment.

140. The method of claim 39, further comprising the step of suggesting at least partially only operating the further body temperature adaption device (300) during time period(s) the infusion device (200) would operate or is operating with the first or second control sensitivity (S200-1, S200-2) in the event that the estimated volume (Vest) does not exceed the first estimated volume portion (Vest 1).
E.g., cooling with infusion fluid is preferred; however pad heating and cooling is required to keep the estimated volume Vest below the maximal volume Vmax.

141. The method of any one of the preceding claims, further comprising the step of at least operating the infusion device (200) if the actual body temperature (Tb act) is above the first upper threshold thereby cooling the patient.

142. The method of any one of the preceding claims, further comprising the step of at least operating the further adaption device (300) if the actual body temperature (Tb act) is below the first lower threshold thereby heating the patient.

143. The method of any one of the preceding claims, further comprising the step of estimating a volume of cooling fluid (Vest cool) to be infused required for cooling the patient during the treatment, and/or
estimating a volume of heating fluid (Vest heating) to be infused required for heating the patient during the treatment.

144. The method of claim 143, wherein the volume of cooling fluid (Vest cool) and the volume of heating fluid (Vest heating) amount for the estimated volume (Vest).

145. The method of claim 144, further comprising the step of suggesting at least partially only operating and/or at least partially only operating the further body temperature adaption device (300) if the actual body temperature (Tb act) is below the first lower threshold, e.g. heating periods in the event that the estimated volume (Vest) exceeds the maximal volume (Vmax) to thereby change the estimated volume (Vest) such that it is equal or below the maximal volume (Vmax).

E.g., the controller may operate at least partially only pad 300 during heating time periods of an induced cooling treatment.

146. The method of any one of claims 141 to 145, further comprising the step of suggesting operating and/or operating at least the infusion device (200) if the actual body temperature (Tb act) is above the first upper threshold, e.g. cooling periods, in the event that the estimated volume (Vest) is within the range defined by and including the volume of cooling fluid (Vest cool) and the maximal volume (Vmax).

E.g., the controller may suggest cooling or cools with infusion fluid if sufficient fluid is to be infused while using pad 300 for heating of an induced cooling treatment.

147. The method of any one of claims 141 to 144, further comprising the step of suggesting at least partially only operating or at least partially only operating the further body temperature adaption device (300) if the actual body temperature (Tb act) is not within the first target temperature tolerance range in the event that the estimated volume (Vest) does not exceed the first estimated volume portion (Vest 1).

148. The method of any one of the preceding claims, further comprising the step of suggesting dividing the treatment into a plurality of treatment phases, the phases comprising at least one of

- a cooling phase (C) for cooling down the patient to a target treatment body temperature (Tb tar treat);
- a holding phase (H) during which the target treatment body temperature (Tb tar treat) is constant; and/or
- a warming phase (W) for re-warming the patient to a desired target body temperature Tb tar end at the end of treatment.

149. The method of claim 148, further comprising the step of deriving for each phase (C, H, W) at least one of the following parameters:

- an estimated volume (Vest C,H,W) to be infused during the respective phase (C, H, W);
- a minimal volume (Vmin C,H,W) to be infused during the respective phase (C, H, W);
- a maximal volume (Vmax C,H,W) to be infused during the respective phase (C, H, W);
- a first lower threshold being lower than the target body temperature (Tbtar) for the respective phase (C, H, W);
- a first upper threshold being higher than the target body temperature (Tb tar) for the respective phase (C, H, W);
- a second lower threshold being lower than the first lower threshold for the respective phase (C, H, W);
- a second upper threshold being higher than the first upper threshold for the respective phase (C, H, W);
- first and/or second target temperature tolerance range(s) defined by lower and upper threshold(s) for the respective phase (C, H, W);
- a control sensitivity (S200 C,H,W, S300 C,H,W) of the infusion device (200) and/or of the further body temperature adaption device (300) for the respective phase (C, H, W);
- a first estimated volume portion (Vest 1) of fluid to be infused with a first control sensitivity (S200-1 C,H,W) of the infusion device (200) for the respective phase (C, H, W); and/or
- a second estimated volume portion (Vest 2 C,H,W) of fluid to be infused with a second control sensitivity (S200-2 C,H,W) of the infusion device (200) for the respective phase (C, H, W), the first control sensitivity (S200-1 C,H,W) being higher than the second control sensitivity (S200-2 C,H,W).

150. The method of claim 149, wherein the minimal volume (Vmin C,H,W) and/or the maximal volume (Vmax C,H,W) to be infused of at least one of the phases (C, H, W) is pre-set by the user.

151. The method of claim 149 or 150, further comprising the step of operating the infusion device (200) and/or the further body temperature adaption device (300) under consideration of a minimal volume (Vmin C,H,W) and a maximal volume (Vmax C,H,W) of at least one of the phases (C, H, W).

152. The method according to any one of claims 149 to 151, further comprising the step of prioritizing the infusion in one of the phases (C, H, W) over others.

153. The method according to any one of claims 149 to 152, wherein the infusion device (200) at least operates during the cooling phase (C) for cooling down the patient, preferably together with the at least one further temperature adaption device (300).

154. The method of any one of the preceding claims, further comprising the step of estimating a temperature adaption rate (R200, R300) of the infusion device (200) and/or of the further body temperature adaption device (300).

155. The method of any one of the preceding claims, further comprising the step of estimating a temperature adaption rate for increasing the body temperature (R200 heat, R300 heat) of the infusion device (200) and/or of the further body temperature adaption device (300).

156. The method of any one of the preceding claims, further comprising the step of estimating a temperature adaption rate for lowering the body temperature (R200 cool, R300 cool) of the infusion device (200) and/or of the further body temperature adaption device (300).

157. The method of any one of the preceding claims 154 to 156, further comprising the step of controlling the infusion device (200) and/or the further body temperature adaption device (300) under consideration of the temperature adaption rate (R200, R300) of the infusion device (200) and/or of the further body temperature adaption device (300).

158. The method of any one of the preceding claims 154 to 157, further comprising the step of controlling the infusion device (200) and the further body temperature adaption device (300) under consideration of their respective temperature adaption rates for increasing the body temperature (R200 heat, R300 heat) and for lowering the body temperature (R200 cool, R300 cool).

159. The method of any one of the preceding claims 154 to 158, further comprising the step of comparing the temperature adaption rates (R200, R300) of the infusion device (200) and the further body temperature adaption device (300), and further comprising the step of selectively operate the one of the infusion device (200) and the further body temperature adaption device (300) having the higher temperature adaption rate (R200, R300), preferably under consideration of a minimal volume (Vmin) and a maximal volume (Vmax) to be applied to the patient during the target treatment time (ttreatment), most preferably so that the estimated volume (Vest) is within the range defined by and including the minimal volume (Vmin) and the maximal volume (Vmax).

160. The method of any one of the preceding claims, wherein the comparison between temperature adaption rates (R200, R300) of the infusion device (200) and the further body temperature adaption device (300) is carried out for increasing the body temperature (R200 heat, R300 heat) and for lowering the body temperature (R200 cool, R300 cool).

161. The method of any one of the preceding claims, further comprising the step of changing or suggesting to change the temperature adaption rate (R200, R300) of the infusion device (200) and/or of the further body temperature adaption device (300), preferably under consideration of a minimal volume (Vmin) and a maximal volume (Vmax) to be applied to the patient during the target treatment time (ttreatment), most preferably so that the estimated volume (Vest) is within the range defined by and including the minimal volume (Vmin) and the maximal volume (Vmax).

162. The method of any one of the preceding claims, further comprising the step of prioritizing the control of the infusion device (200) over the further body temperature adaption device (300) and/or of the further body temperature adaption device (300) over the infusion device (200).

163. The method of any one of the preceding claims, further comprising the step of operating in different treatment modes.

164. The method of claim 63, further comprising the step of varying the control accuracy of the actual body temperature (Tb act) during the treatment time depending on the treatment mode.

165. The method of claim 64, wherein the control accuracy is varied by varying the first and/or second target temperature tolerance range(s).

166. The method of claim 164 or1 65, wherein the control accuracy is varied by varying the sensitivity of the infusion device (200) and/or the further body temperature adaption device (300).

167. The method of any one of the preceding claims 164 to 166, wherein in a modus of treatment with high control accuracy the body temperature is controlled by operating at least the infusion device (200), and wherein in a modus of low control accuracy the body temperature preferably predominantly is controlled by operating the further body temperature adaption device (300), and wherein the control accuracy during the period of treatment with high control accuracy is higher than during the period of low control accuracy.

168. The method of any one of the preceding claims, further comprising the step of checking prior to operation of at least one of the at least two different body temperature adaption device (200, 300), whether the estimated volume (Vest) is within the range defined by and including the minimal volume ($V_{min}$) and the maximal volume ($V_{max}$).

169. The method of any one of the preceding claims, further comprising the step of estimating the estimated volume ($V_{est}$) during the operation of the method.

170. The method of any one of the preceding claims, further comprising the step of changing operation of at least one of the at least two different body temperature adaption device (200, 300), if the estimated volume ($V_{est}$) is not within the range defined by and including the minimal volume ($V_{min}$) and the maximal volume ($V_{max}$).

The disclosed apparatus for controlling a body temperature $T_b$, may comprise a controller. The controller may be adapted to control the body temperature $T_b$, by controlling at least two different body temperature adaption means or body temperature adaption devices. Preferably, one body temperature adaption device is an infusion means or infusion device. Preferably, the at least one further body temperature adaption device is different from the one body temperature adaption device and, more preferably, not an infusion device. The infusion device preferably encompasses blood/blood derivates and/or fluid infusion systems and/or preferably is a infusion system for infusing, e.g., saline or other balanced fluids like ringer's solution.

The infusion device is adapted to add a volume of fluid to the patient's blood circuit, the volume of fluid may have a different temperature than the body temperature $T_b$,, more specifically than the blood of the body, thereby adapting the body temperature. The mixing of blood and added fluid results in a change in temperature of the patient's blood and thus of the body temperature. The infusion device may be an apparatus to provide the infused fluid. The infusion device may be adapted to change the volume flow and/or the temperature of the fluid. Such devices are disclosed in for instance in patent application EP-A- 2 514 453. EP-A-2 514 453 disclosed an apparatus considering the actual temperature and at least one additional parameter representing the physiological state of the patient. The disclosure of this apparatus considering above parameters is incorporated by reference in this application. WO2009056640 A3 discloses the volume and/or temperature control of the fluid to be infused by an infusion device. The volume and/or temperature control of the fluid to be infused is herewith incorporated by reference. EP-A-2698182 discloses a particular way adjusting the temperature of medical liquids to be infused by the infusion device. Also method as specified in claim 1 of this document as well as the relevant parts of the description is herewith incorporated by reference.

The at least one further body temperature adaption device may be a volume neutral device or means. In other word, the at least one further body temperature adaption device is adapted to change the body temperature without changing the blood-volume. The blood volume is to be considered as the fluid volume in the vessels of the patient. Volume neutral means that the device does not provide an additional liquid volume to the patient while adapting the temperature of the patient. The at least one further adaption device may be a heat exchange based device. The heat exchange may happen at skin, in a hollow organ, and/or in a body cavity incl. blood vessels, etc. The heat exchange may be based on a heat transfer, e.g. by convection and/or by conduction. E.g. the device may interact with a surface of the patient such as the skin of a patient or an intravascular catheter may comprise a heat exchanger exchanging heat with the blood flowing around the catheter. In the context of the present application heat exchange does not include mixing of fluid which is considered to take place when an infusion means is used.

Heat exchanged based temperature adaption devices may comprise at least one of: temperature adaption pad(s), preferably applied to the patient's skin,, coolings vests, head wraps, intravascular catheter(s), gas inhalation, transnasal evaporative catheter systems extra-corporal adaption of the blood temperature, temperature adaption mattress and/or blankets, temperature adaption tents, heart-lung machine, peritoneal-lavage systems, blood and fluid warmers etc.

The controller may be adapted to control the body temperature $T_b$, by simultaneously controlling the infusion device and the at least one further body temperature adaption device. The controller is preferably adapted to selectively or simultaneously operate the infusion device and the at least one further body temperature adaption device.

Operating the infusion device and/or the further body temperature adaptation device involves adjusting one or more parameters of said means. The parameters of the infusion device are the infusion parameters, e.g. temperature and/or volume flow of the liquid to be infused. A parameter of the at least one further temperature adaption device is, for instance, the surface temperature of the heat exchanger. This surface temperature might be indirectly controlled, e.g. via the fluid flow in heat exchanger. Another parameter might be the involved/active surface of the further temperature adaption device. The adaption of the parameters could also be done in cooperation with further controller(s) of the infusion device and/or of the further body temperature adaptation device. In other words, the controller has the capacity to continuously control the operation of the at least two temperature adaption devices by providing control signals to the devices. Even though the controller may choose to selectively operate only one of the temperature adaption devices, it may be configured to control at the same time both devices. The control signal may be any kind of suitable signal so that the actuators of the at least two temperature adaption means operate. The controller may operate the infusion device and/or the at least one further body temperature adaption device to keep the actual body temperature $T_{b\ act}$, close to a target or nominal or setpoint body temperature $T_{b\ tar}$.

The controller may be adapted to operate the infusion device and/or the further body temperature adaption device under consideration of a minimal volume and a maximal volume to be applied to the patient during the target treatment time. The controller may adapt the temperature by using the infusion device or the further means depending what is deemed appropriate. E.g. when infusion volume is critical and the further adaption device is deemed appropriate the further adaption device may at least predominantly be used. In doing so, the volume might be kept below a critical level and the patient may suffer less. If the infusion volume is close to the maximal volume $V_{max}$ even the emergency stop of the treatment might be avoided. The minimal volume $V_{min}$ and/or the maximal volume $V_{max}$ may be defined by the user, preferably during set up. Alternatively or additionally the minimal volume $V_{min}$ and/or the maximal volume $V_{max}$ may be preset, e.g. by the machine maker or other skilled personnel, such as a physician. The values for the minimal volume $V_{min}$ and/or $V_{max}$ may vary or may be varied during the treatment period.

The controller is adapted to estimate or to calculate or to derive an estimated volume $V_{est}$ to be applied/infused to the patient during the overall or remaining target treatment time $t_{treatment}$. That is, the controller may be adapted to estimate the estimated volume prior to treatment for the entire desired temperature profile as further discussed below during the overall target treatment time. The controller is also adapted to estimate or update the estimate for the estimated volume $V_{est}$ during the course of the therapy, for instance in the event that the initial estimate is not in line with the patient's response to the therapy resulting in a difference between actual volume infused and estimated volume until then. The estimate may be based on one or more, preferably all of the parameters: selected treatment modus, weight of the patient, temperature of the fluid to be infused, body-mass-index. Eventually, also the, target body temperature profile may form basis for the estimate. Prior to treatment, the controller may estimate an initial estimated volume $V_{est}$ using the target or assumed initial values of aforementioned parameters. After start of the treatment, the controller may be adapted to compare the actual values of the parameters as well as of the estimated volume with the initially or presently estimated values. If a (significant) difference occurs, e.g. due to the patient's reaction on the therapy and/or due to user input, the controller may update/adapt the estimated volume. Preferably, the controller is adapted to update the estimates periodically, more preferably on an ongoing basis. The controller may be adapted to suggest and/or derive a control signal from the updated estimated values. That is, the controller may control the at least two different body temperature adaption devices based on at least one updated estimated value of the estimated volume or a parameter influencing the estimated volume. The apparatus is adapted that the operation of the controller as well as the estimated values derived by the controller may be overruled by a user input. For instance, the user may define a minimum volume to be infused in order to improve the general physiological state of the patient. E.g. a minimal volume may be required with patient's having suffered blood losses, while the effective maximal volume to be applied may by varied by the clinical staff depending on the degree of injuries. The estimated values such as the estimated volume to be infused may be derived from empirical data gained form clinical studies, e.g. with patients with different weight, BMI, temperature of the infused fluid and different target temperature profiles. For instance the estimated values may be stored in a memory. The controller may be adapted to look up the estimated values in a look up table or data field, e.g. configured as a multi-dimensional matrix. The user may then enter the specific values of the patient and the desired treatment profile and the corresponding $V_{est}$ will be looked up in a table/matrix. For instance, as a general rule, a temperature adaption rate for cooling up to 3,2°C/h may be obtained when infusing approximately 30 mg/kg/h of infusion fluid being about 4° cold. Similar empirical relationships could be approximated for holding a patient at a specific target temperature and for bringing a patient back to the normal body temperature at the end of a treatment.

The controller may be adapted to estimate a temperature adaption rate of the infusion device and/or of the further body temperature adaption device. The temperature adaption rate may be estimated based on user input, e.g. the desired settings of the infusion device (e.g. fluid temperature) and/or the at least one further temperature adaption device (e.g. surface temperature of pad), and/or based on machine core data, e.g. by using a lookup table for known products on the market. The temperature adaption rate is a measure of the responsiveness of each of the at least two different body temperature adaption devices. A high temperature adaption rate means a quick control response to a change of the actual body temperature. It could be described by the formula:

$$(1) \qquad R = DELTA\ T_{b\ act} / DELTA\ t_{treatment}$$

wherein:

R                   is the temperature adaption rate

DELTA $T_{b\ act}$     is the actual change of body temperature, and

DELTA $t_{treatment}$   is the period of time during which the change of body temperature occurred

Alternatively, the user may manually input the temperature adaption rates or values may be pre-set by the maker. The controller is adapted to estimate a temperature adaption rate for lowering the body temperature of the at least two body temperature adaption device. For instance, the temperature adaption rate for cooling might be substantially higher than for warming with the infusion means. The controller may be adapted to change or to suggest to change the temperature adaption rate of the infusion device and/or of the further body temperature adaption device, preferably under consideration of a minimal volume $V_{min}$ and a maximal volume ($V_{max}$ to be applied to the patient during the target treatment time $t_{treatment}$, most preferably so that the estimated volume $V_{est}$ is within the range defined by and including the minimal volume $V_{min}$ and the maximal volume $V_{max}$. For instance, the adaption rate may be changed by changing the fluid temperature and/or the temperature of the surface of the pad. There may be two levers to optimize volume while having a good control quality:

1. use the temperature adaption device with the best adaption rate, and
2. if required or desired: adapt the adaption rate of the temperature adaption device.

The present invention will become more fully understood from the detailed description given hereinafter and the accompanying drawings which are given by way of illustration only, and thus, are not limitative of the present invention, and wherein:

Fig. 1     is a schematic drawing of the apparatus with the controller 100;
Fig. 2     is a graph showing the target body temperature profile $T_{b\ tar\ prof}$;
Fig. 3     is a graph showing the course of the actual body temperature $T_{b\ act}$ during the therapy;
Fig. 4     is a graph showing the course of the actual body temperature $T_{b\ act}$ during the cooling phase C depicted in Fig. 3;
Fig. 5     is a graph showing the course of the actual body temperature $T_{b\ act}$ during the maintaining phase H depicted in Fig. 3;
Fig. 6     is a graph showing the course of the actual body temperature $T_{b\ act}$ during the rewarming phase W depicted in Fig. 3;
Fig. 7     is a flow diagram depicting the process during setup and/or operation;
Fig. 8     is a flow diagram depicting the process during the volume optimization step S30;
Fig. 9     depicts the influence of the process step S331, S333 or S334 on the infusion volume;
Fig. 10    depicts the influence of the process step S332 on the infusion volume;
Fig. 11    depicts the influence of the process step S333, S334 or S335 on the infusion volume;
Fig. 12    depicts the influence of the process step S335 on the infusion volume;
Fig. 13    shows a diagram indication the distribution of the estimated volume;
Fig. 14    is a schematic drawing of the apparatus with shared components; and
Fig. 15    is a schematic drawing of the apparatus with shared components.

While the figures depict a temperature profile for induced cooling, it is noted that the same effects generally apply to induced heating. The further discussion also encompassed induced heating, even if cooling is explicitly mentioned.

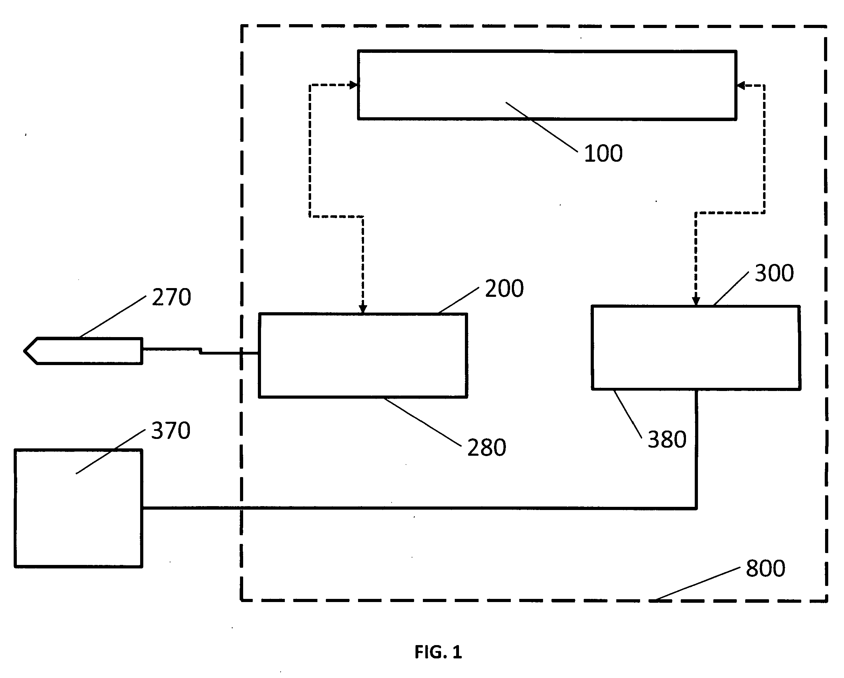

Fig. 1 is a schematic drawing of the apparatus with the controller 100. Controller 100 is here the common controller 100 of infusion device 200 and the at least one further body temperature adaption means 300, here configured as a temperature adaption pad comprising a housing 380 and the pad 370. The infusion device 200 may comprise an infusion device housing 280 and an infusion needle 270 fluidly connected with the infusion device housing 280. Further components of the infusion device 200 as well as of the pad 300 are not shown here. The controller 100 may be adapted to provide control signals, here indicated by dotted lines to the infusion device 200 as well as to the pad 300. Infusion housing 280 and housing 380 may be located in the housing 800 of the apparatus. However, it is noted that infusion device 200 and/or the at least one further body temperature adaption means 300 may not be provided in the housing of the apparatus 800 as long as they are operationally connected with the controller 100, e.g. wired and/or wireless. Alternatively, the controller 100 may be located in the infusion housing 280 or in the housing of the pad 300. Infusion device 200 and/or the at least one further body temperature adaption means 300 may have sub-controller, preferably adapted to at least assisting to control the body temperature.

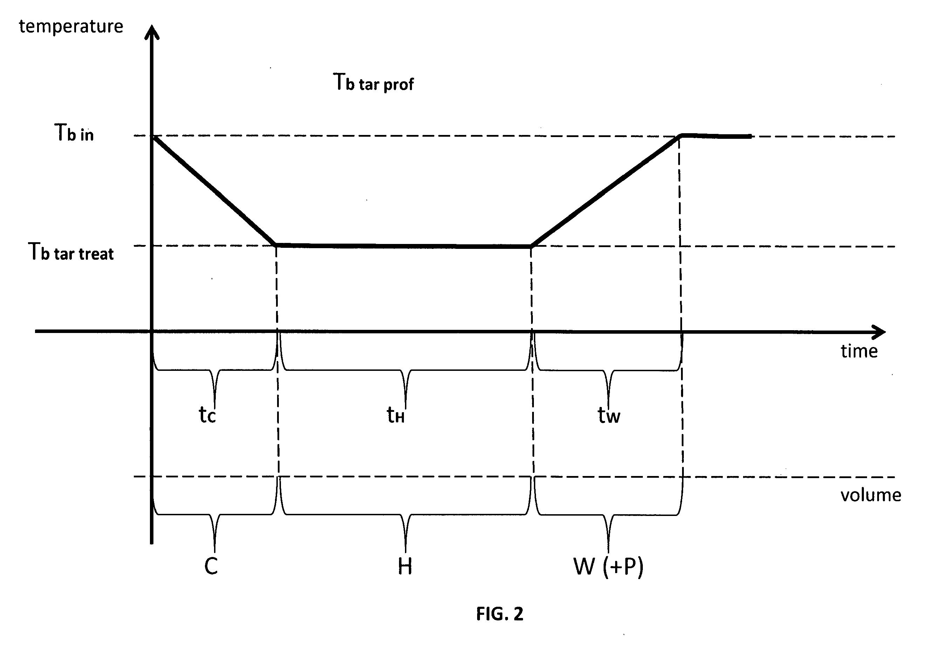

Fig. 2 depicts a target body temperature profile $T_{b\ tar\ prof}$ versus time. The target body temperature profile $T_{b\ tar\ prof}$ may be considered as the course of the desired target temperature. Here, the profile of the desired target body temperature $T_{b\ tar}$ over the time from start of the treatment until the end; i.e. over the treatment time $t_{treatment}$, is shown.

The course of the body temperature, course of the body temperature over the (treatment) time, and the body temperature profile are to be considered as synonyms for the respective target or actual values of the body temperature. Figure 1 depicts a typical treatment arrangement for induced cooling. For instance, the patient initially may have an initial actual body temperature $T_{b\,in}$ of 37°C. The cooling starts at t=0 seconds with cooling down the patient from the normal or initial body temperature $T_{b\,in}$ to the desired target treatment body temperature $T_{b\,tar\,treat}$. After a first or cooling down time period $t_c$ the patient is cooled down to the target treatment body temperature $T_{b\,tar\,treat}$, for instance 34°C. This target temperature is maintained for the time period $t_H$, for instance, 5 or 10 hours, preferably with a tolerance within a specified tolerance range, e.g. of about 0,3°C. The temperature may be held relatively constant by using a closed loop control. The controller may be adapted to infuse cold or warm fluid, preferably intermittently. The patient is then re-warmed back to a target body temperature $T_{b\,tar\,end}$ at the end of treatment, which is here the same temperature as the initial body temperature $T_{b\,in}$, for instance, of 37°C, during a second or rewarming time period $t_W$, e.g. 3 hours. The treatment may be divided into a plurality of treatment phases C, H, W. The phases may be, for instance:

- a cooling phase C for cooling down the patient to a target treatment body temperature $T_{b\,tar\,treat}$;
- a holding phase H during which the target treatment body temperature $T_{b\,tar}$ is about the same as the target treatment body temperature $T_{b\,tar\,treat}$; and/or
- a warming phase W for re-warming the patient to a desired temperature.

Parameters and values comprising a suffix such as "C", "H", and "W" refer to parameters and values of or defined for the respective treatment phase C, H, W. The total treatment time may be calculated as:

$$(2) \qquad t_{treatment} = t_c + t_H + t_W$$

wherein:

$t_c$     is the cooling down time period;
$t_H$     is the time period, wherein the body temperature is maintained at a preferably relatively constant temperature; and
$t_W$     is the re-warming time period.

The maximal volume to be infused during the treatment may be calculated with the formula:

$$(3) \qquad V_{max} = V_c + V_H + V_W + P$$

wherein:

$V_c$     is the volume of infusion fluid required to cool down the patient to the target temperature;
$V_H$     is the volume of infusion fluid required to maintain the body temperature at a the desired preferably relatively constant target temperature; and
$V_W$     is the volume of infusion fluid required to re-warm the patient to a desired body temperature at the end of treatment (for instance 37°C), and
$V_P$     is a buffer or supplement volume

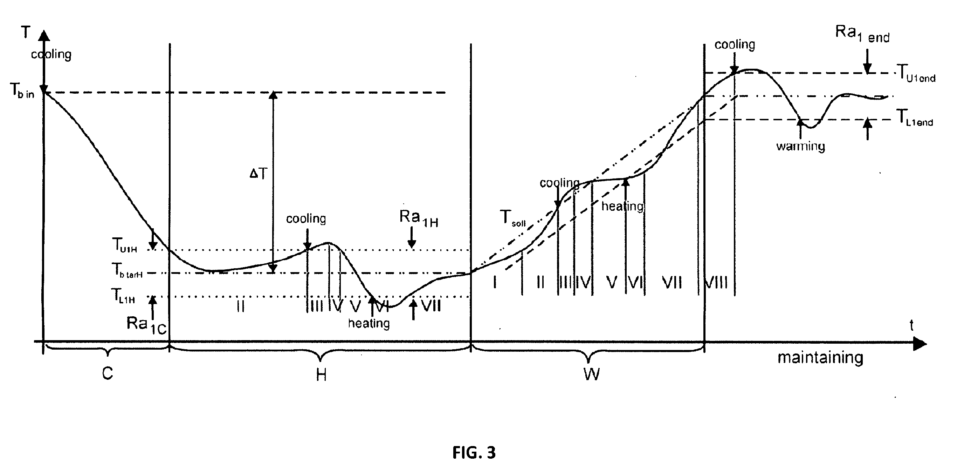

The maximal volume $V_{max}$ is often described as a maximal volume per day, e.g. 6 liter per day. The supplement volume $V_P$ is preferably used in cases where the re-warming is too fast. The patient may then be cooled with infusion fluid without exceeding the maximal volume $V_{max}$. Preferably, the supplement volume is infusion fluid being at the lowest possible temperature, generally about 4°C. The value of the supplement volume $V_P$ may be a preset value, preferably a preset machine value. The supplement value $V_P$ may be used to avoid or reduce overshooting of the body temperature of the patient. Overshooting means that the body temperature of the patient at the end of the treatment is getting higher than the intended target temperature towards the end of treatment (see Fig. 3). Overshooting is linked to severe negative effects and may deteriorate the treatment result of induced cooling. The controller may be adapted to estimate at least one of the following parameters: estimated volume $V_{est}$ required for the treatment, minimal estimated volume $V_{min\,est}$, maximal volume $V_{max\,est}$, $V_{est\,t}$ estimated volume up to a time t. The controller may be adapted to estimate further parameters indicated with the suffix "est".

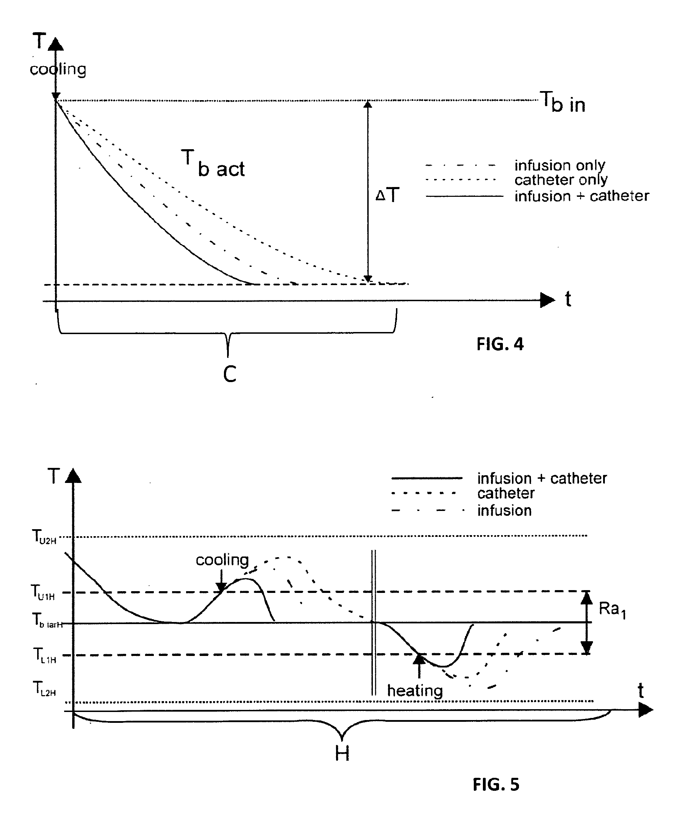

As depicted in Figs. 3 and 4, during the cooling phase C the controller 100 may control the at least two different body temperature adaption devices 200, 300 so that both preferably operate with the maximal temperature adaption (i.e. cooling) rate. The controller 100 is here preferably configured not to control the actual body temperature ($T_{b\,act}$) to be within a target tolerance range $Ra_{1C}$. Instead controller 100 is operating the at least two different body temperature adaption devices 200, 300 so as to continuously cool the patient, preferably at the maximal rates. By not defining a target tolerance range $Ra_{1C}$ the controller is prevented from intermittent operation further explained below. The controller may be adapted to operate the temperature adaption devices so as to cool down the patient as fast as possible. Depicted in Fig. 4 are the different temperature curves for the actual body temperature of the patient. Depicted as a dash-dotted line is the actual body temperature assuming that the patient is only cooled by the infusion device 200. The dotted line shows the temperature curve of the actual body temperature in the event that the body is only cooled using the further body temperature adaption device, here an intravascular catheter 300. The graph shows that the infusion device 200 having a higher temperature adaption rate $R_{200}$ is adapted to cool down the patient quicker than catheter 300 having an adaption rate $R_{300}$ being lower than the rate $R_{200}$ of the infusion device 200. The combination of both the infusion device 200 and the catheter 300 leads to the best overall cooling rate and the shortest cooling down time period $t_C$ required to cool the patient from the initial body temperature $T_{b\,in}$ to a desired target treatment body temperature $T_{b\,tar\,H}$.

As depicted in Figs. 3 and 5, the body temperature may be maintained at a desired target treatment body temperature $T_{b\,tar\,H}$, during the time period $t_H$. More precisely, the body temperature is held within a first temperature tolerance range $Ra_{1H}$ for the time period $t_H$ defined by a respective first upper threshold value $T_{u1H}$ and a respective first lower threshold value $T_{l1H}$. As long as the actual body temperature is within the first temperature tolerance range preferably none of the at least two different temperature adaption devices 200, 300 is operating. If the actual body temperature is not within the first temperature tolerance range $Ra_{1H}$ the controller 100 is actively operating at least one of the at least two different temperature adaption devices 200, 300 in order to shift the actual body temperature back into the first temperature tolerance range $Ra_{1H}$. Similar as in Fig. 4 the dotted line depicts the body temperature curve if only the catheter 300 operates and the dash-dotted line depicts the body temperature curve for infusion device 200 operations only. The operation of both the adaption devices show the best control behaviour ensuring the body temperature achieving a value within the first temperature tolerance range after relatively short times compared to the operation of only one temperature adaption means 200, 300. Also depicted in Fig. 5 is the second lower threshold $T_{l2H}$ being lower than the first lower threshold, and the second upper threshold $T_{u2H}$ being higher than the first upper threshold. Not shown in Fig. 5 is the situation when the actual body temperature is not within a second temperature tolerance range $Ra_{2H}$ of the maintaining time period defined by the respective second lower threshold $T_{l2H}$ and the respective second upper threshold $T_{u2H}$. In that case, the controller may always operate both the infusion means 200 and the catheter 300 in order to bring the actual body temperature quickly back to values within the first temperature tolerance range. The body temperature adaption rate may be restricted to a maximum body temperature adaption rate, for instance of less than 1°C per hour, preferable of less than 0,5°C per hour and most preferably of about 0,3°C per hour.

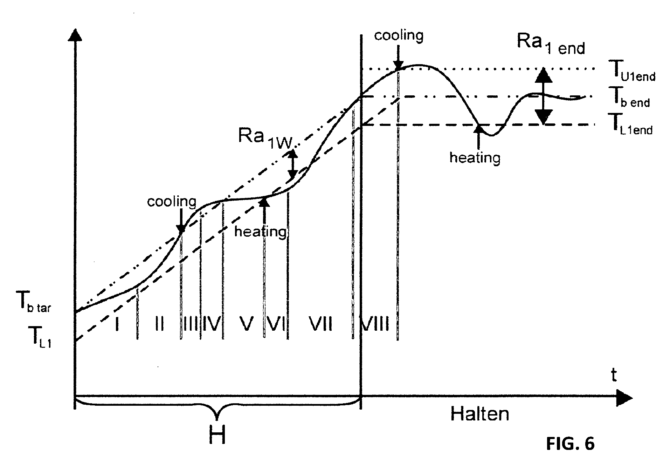

Next, the time period $t_W$ for re-warming is further explained using Figs. 3 and 6. As already indicated above, during re-warming care should be taken that the patient gets not too hot. Any overshooting should be avoided or reduced to a minimum. Therefore, the first and/or second upper threshold value $T_{u1W}$, $T_{u2W}$, for the re-warming phase W may be chosen to be close or equal to the target body temperature $T_{b\,tar\,W}$. That is, a first and/or second temperature tolerance range $Ra_{1W}$. $Ra_{2W}$ may be here encompass a range below the target body temperature $T_{b\,tar\,W}$. The general control behavior and control design is similar to that of the maintaining phase H with the difference that the target body temperature $T_{b\,tar\,W}$ preferably gradually increases towards the end of treatment towards the target body temperature $T_{b\,tar\,end}$.

Thus, generally speaking the target body temperature profile $T_{b\,tar\,prof}$ is a course over the target treatment time which may comprise zones or sections or phases of comparatively increased accuracy or temperature tolerance ranges and zones or sections of comparatively reduced accuracy or temperature tolerance ranges.

Above depicted control design may also be further described by different scenarios indicated in below table 1

Table 1

| Scenario | Area in Figure | Description | Reaction of the controller 100 |
|---|---|---|---|
| $T_{b\,act} < T_{b\,tar}$ and $T_{b\,act} > T_{min}$ and $dT/dt > dT_{b\,tar}1/dt$ | II | approaching the target body temperature curve | no reaction |
| $T_{b\,act} < T_{b\,tar}$ and $T_{b\,act} > T_{min}$ and $dT/dt < dT_{b\,tar}/dt$ | I, V | departing from the target body temperature curve, actual body temperature above lower threshold | no reaction |
| $T_{b\,act} < T_{b\,tar}$ and $T_{b\,act} < T_{min}$ and $dT/dt < dT_{b\,tar}/dt$ | V→VI | departing from the target body temperature curve, actual body temperature below lower threshold | warming |

(continued)

| Scenario | Area in Figure | Description | Reaction of the controller 100 |
|---|---|---|---|
| $T_{b\,act} = T_{b\,tar}$ and $dT/dt = dT_{b\,tar}/dt$ | -- | Actual body temperature in line with target body temperature curve | no reaction |
| $T_{b\,act} = T_{b\,tar}$ and $dT/dt > dT_{b\,tar}/dt$ | II→III | Target body temperature curve crossed from below | cooling |
| $T_{b\,act} > T_{b\,tar}$ and $dT/dt < dT_{b\,tar}/dt$ | IV | Target body temperature curve approached from above | no reaction |
| $T_{b\,act} > T_{b\,tar}$ and $dT/dt > dT_{b\,tar}/dt$ | -- | Departing from the target body temperature curve | cooling |

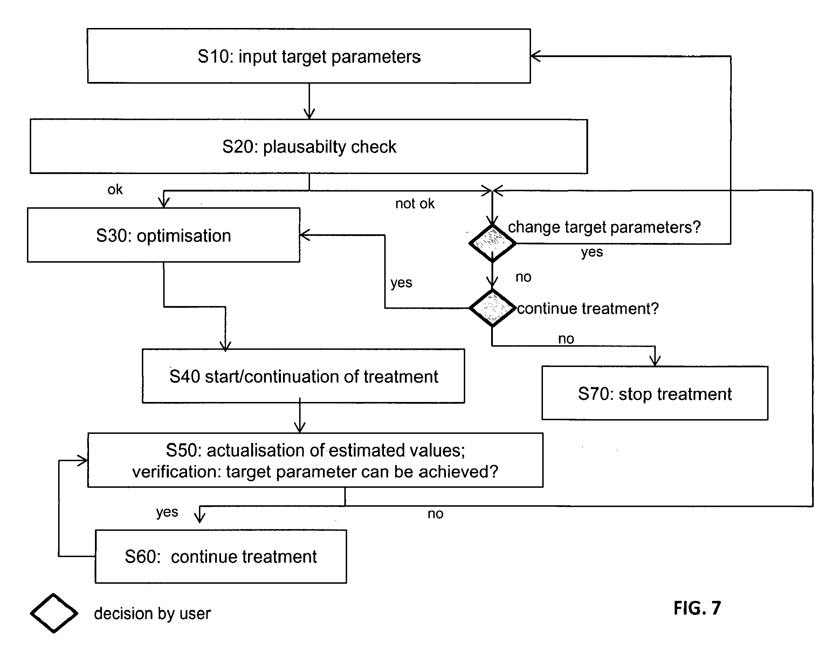

Fig. 7 is a flow diagram depicting the process during setup and/or operation. At step S10 the input parameter may be set. The input parameters may parameters provided by the clinical staff. Preferably, the input parameters may comprise at least one of the following parameters: minimal volume $V_{min}$ and maximal volume ($V_{max}$ of the infusion fluid, body mass index and weight. In addition, parameters describing the desired target body temperature profile $T_{b\,tar\,prof}$ may be used.

The apparatus may be adapted to select or suggest the desired target body temperature profile $T_{b\,tar\,prof}$ based on input parameters comprising desired target treatment body temperature $T_{b\,tar\,treat}$ and the desired total treatment time. The target values for the cooling phase C, the holding phase or maintaining phase H, and/or the warming phase W of the target body temperature profile $T_{b\,tar\,prof}$ may be suggested by the apparatus based on the other input parameters. Alternatively, these values may be chosen by the clinical staff in order to define the desired target body temperature profile $T_{b\,tar\,prof}$. The selection of the target values for the plurality of different phases C, H, W are based on data obtained during clinical studies and may be adapted depending on the physiological condition of the patient.

The clinical staff may select an operation modus of the apparatus. One first modus of treatment may be a modus with high control accuracy. This means that the device is controlling at least in some phases, preferably selected by the clinical staff, the body temperature in a relatively small tolerance range Ra compared to another modus, in which the volume of fluid volume is relatively low. In the modus of increased control accuracy, the controller controls the body temperature by operating at least the infusion device 200. Particularly during induced cooling the infusion device provides a relatively high temperature adaption rate $R_{200}$ and a relatively high control sensitivity. In other words, in this modus the target body temperature profile $T_{b\,tar\,pro}$ should be met as good as possible.

In a second modus which can be selected by the clinical staff, the controller 100 will operate with a relative low control accuracy compared with the first modus of high control accuracy. The controller 100 controls the body temperature preferably predominantly by operating the further body temperature adaption device 300. Since the further body temperature adaption device 300 is infusion volume neutral, the volume of infusion fluid to be infused may be relatively low compared to the first modus. On the other hand, the device 300 provides a relatively low temperature adaption rate $R_{300}$ and a relatively low control sensitivity compared to the values of the infusion device 200. Therefore, the control accuracy of the second modus is lower compared with the first modus.

At step S20 depicted in Fig. 7 the controller 100 verifies the input data provided by the clinical staff. The controller may estimate the volume $V_{C\,est}$, $V_{H\,est}$, $V_{W\,est}$, required for each of the plurality of phases C, H, W. The values are estimated based on data obtained during clinical research and stored in the apparatus as discussed above for the estimation of $V_{est}$. The output of the plausibility check will be provided to the clinical staff, for instance displayed on a human machine interface.

If the maximal volume ($V_{max}$ is less than the estimated volume $V_{C\,est}$ to be infused during the cooling time period the may verify whether the further temperature adaption means 300 may provide the desired target body temperature profile $T_{b\,tar\,prof}$. The apparatus may then provide a feedback that the treatment may not be achieved by infusion. Moreover, the controller may inform whether the further temperature adaption means 300 would be suitable.

If the maximal volume $V_{max}$ is greater than the estimated volume $V_{C\,est}$ and if the sum of the estimated volumes $V_{C\,est}$ and $V_{H\,est}$ to be infused during the cooling and maintaining periods is greater than the maximal volume $V_{max}$ then an adaption of the target parameters may be required. The apparatus may provide a warning that the patient may be cooled down by infusing but the target treatment body temperature may not be maintained during the entire time period the patient should be maintained at that temperature. Re-warming with infusion may not be possible. The apparatus may verify whether the volume to be infused may be optimized and/or may suggest optimizing the volume to be infused.

If the maximal volume $V_{max}$ is greater than the sum of the estimated volumes $V_{C\,est}$, $V_{H\,est}$ and $V_{W\,est}$ and if the maximal volume $V_{max}$ is less than the sum of the estimated volumes $V_{C\,est}$, $V_{H\,est}$, $V_{W\,est}$ and the buffer P also then an adaption of the target parameters may be required. The apparatus may verify whether the volume to be infused may be optimized and/or may suggest optimizing the volume to be infused.

If the maximal volume $V_{max}$ is greater than the estimated volume for the treatment which is the sum of the estimated volumes $V_{C\ est}$, $V_{H\ est}$, $V_{W\ est}$ and the buffer P, then the treatment can be started without delay. The volume to be infused is estimated not to be critical and no complications are expected. Further adaption of the target values may be possible but are not required. The apparatus may verify whether the volume to be infused may be optimized and/or may suggest optimizing the volume to be infused, for instance if the clinical staff desired to optimize the volume to be infused.

During step S20 the controller verifies whether the desired target body temperature profile $T_{b\ tar\ pro}$ will be achieved with the present settings and/or whether an optimization is required. If the desired target body temperature profile may be achieved with the present settings ($V_{max} > V_{est}$) then the controller may continue directly with step S40. If the controller 100 during step S20 determines that an optimization of the volume to be infused is required and/or if the clinical staff desires, the optimization of the volume to be infused may be carried out in step S30. If the controller 100 determines that the desired target body temperature profile $T_{b\ tar\ prof}$ may not be achieved with the preselected parameter setting then the controller may suggest changing the parameter settings. The clinical staff then may decide whether to change at least one of the target parameters (as shown in Fig. 7). If the clinical staff has decided to change at least one of the target parameters, then the controller may continue with step 10 and/or step 20.

If the parameter setting is considered as not being suitable to carry out the treatment in accordance with the desired target body temperature profile and if the clinical staff has decided not to change at least one of the target parameters, the controller may ask the clinical staff whether the treatment should be continued anyway. If the clinical staff decides not to carry out the treatment then the procedure is stopped. If the clinical staff decides to start the treatment with the parameter set considered as not being suitable for achieving the desired target body temperature then the controller may be adapted to carry out the optimization of the volume defined in step S30. After the volume optimization step S30 is carried out, the controller will start the treatment at step S40.

Once the treatment is started, controller 100 verifies intermittently or continuously whether the actual values/parameters of the treatment are in line with the estimated values/parameters. For instance, the controller may compare the actual volume already infused to a patient with the estimated volume to be infused up to that given time. Similarly, also the estimated values for each of the plurality of treatment phases may be compared to the respective actual values. If the estimated values differ from the measured actual values, then the estimated values may be changed based on the data available from clinical studies stored in the apparatus and the measured actual values. The controller may then be adapted to verify whether a treatment in accordance with the defined target body temperature profile may be achieved with the changed estimated values. If the defined target body temperature profile may be achieved with the changed estimated values obtained during step S50, then the treatment will be continued without a change in the settings of the target parameter (step S60) while continuously carrying out step S50.

If the defined target body temperature profile will not be achieved with the changed estimated values obtained during step S50, then the controller may ask the clinical staff to change the target parameter or may suggest changed target parameters. The clinical staff may then decide to update the target parameters. If the clinical staff decides to update the target parameter, then the controller may continue with steps S10 and S20 explained above. If the clinical staff decides not to change the target parameter, then the controller may request the clinical staff to advise whether the treatment should be continued with the present target parameters or not. If the clinical staff decides not to continue the treatment, then the treatment will be stopped in step S70. Otherwise, a controller may again carry out the infusion volume optimization step S30. The treatment will be continued (step S40).

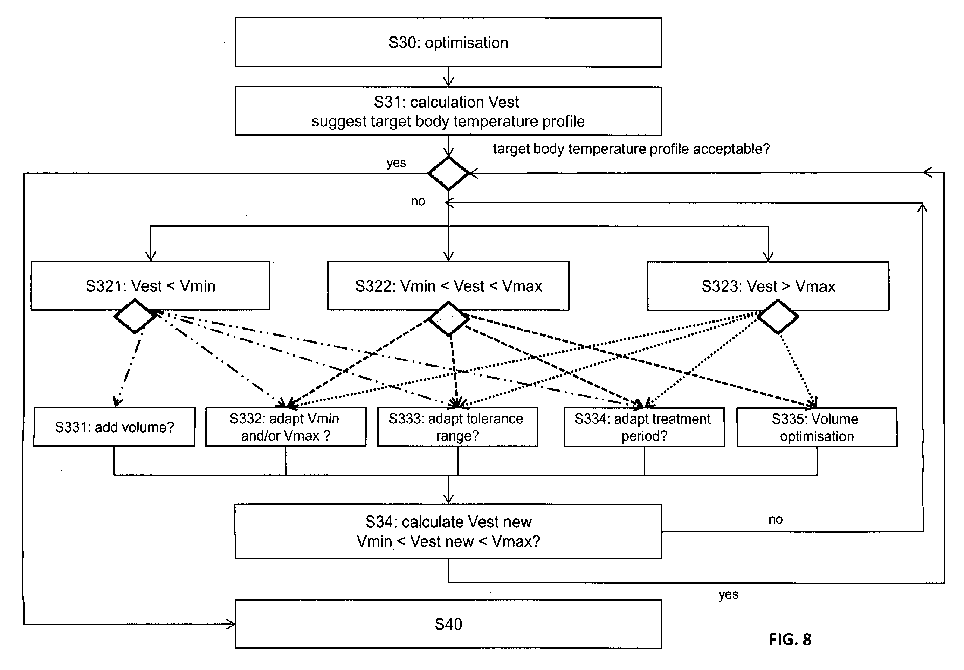

Figure 8 is a flowchart depicting the process during volume optimization step S30. In step 31 the estimated volume $V_{est}$ may be calculated. The controller may be adapted to suggest a target body temperature profile during step S31. If the suggested target body temperature profile is acceptable, then the controller will start or continue the treatment (step S40). If the target body temperature profile is not acceptable, then the controller may compare the estimated volume to be infused with the minimal volume $V_{min}$ and/or with the maximal volume $V_{max}$. If the estimated volume $V_{est}$ is less than the minimal volume $V_{min}$ (S321) then the controller may be adapted to suggest and/or to carry out at least one of the steps S331 to S334 further explained below. If the estimated volume $V_{est}$ is greater than the minimal volume $V_{min}$ and if the estimated volume $V_{est}$ is less than the maximal volume ($V_{max}$ then the controller may be adapted to suggest and/or carry out at least one of the steps S332 to S335 discussed below. If the estimated volume $V_{est}$ is greater than the maximal volume ($V_{max}$ than the controller 100 may be adapted to carry out at least one of the steps S332 to S335. The measures defined in steps S331 to S335 may also simultaneously be applied. The measures of steps S331 to S335 may result in a change in at least one of the target parameters.

In step S34 the controller may calculate the changed estimated volume $V_{est}$ new based on the changed target parameter(s). The controller 100 may verify in step S34 whether the changed estimated volume $V_{est}$ new is within the range defined by and including the minimal volume $V_{min}$ and the maximal volume $V_{max}$. If $V_{est}$ new is within said range, then the controller 100 may suggest the resulting (eventually) new target body profile to the clinical staff. If the target body temperature profile is acceptable, then the therapy may be started or continued with the (newly) defined target body temperature profile (step S40). If the profile is deemed not acceptable by the clinical staff, then the controller continues with step S322. If the changed estimated volume $V_{est}$ new is not within the range defined by and including the minimal volume $V_{min}$ and the maximal volume ($V_{max}$ then the controller may continue with the steps S321 or S323. Steps S331 to S335 are volume adaption measure. The controller 100 may be adapted to suggest and/or change to a new target body temperature profile $T_{b\ tar\ prof\ new}$ based on one or more, preferably all of the parameters: treatment mode, weight of the patient, temperature of the fluid to be infused, body-mass-index, desired target body temperature profile $T_{b\ tar\ prof}$ desired.

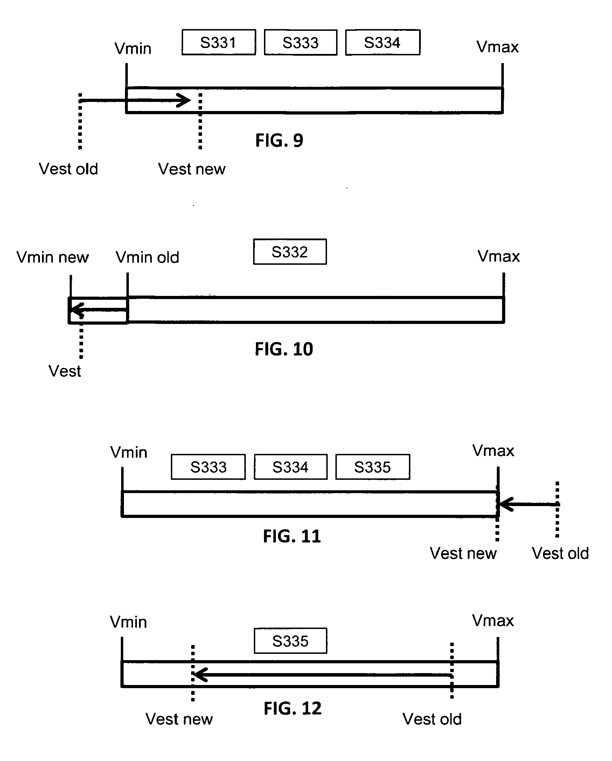

As show in Fig. 9 the controller 100 may be adapted to change volume flow and/or a temperature of the infused fluid in the event that the estimated volume $V_{est}$ is not within the range defined by and including the minimal volume $V_{min}$ and the maximal volume $V_{max}$. For instance, as depicted in step S331 more volume of less cold fluid may be infused in the event of induced cooling or alternatively, the infusion of fluid with the same temperature (e.g. 4°C) will be continued and additionally a determined fluid flow with about body temperature will be infused. By increasing the volume flow the estimated volume will be shifted from below $V_{min}$ to a value within the range defined by $V_{min}$ and $V_{max}$. Inversely, also a lowered volume flow with a decreased fluid temperature may be applied in order to shift estimated volume ($V_{est}$) below the maximal volume ($V_{max}$) in the event of induced cooling.

According to step S332 the controller is adapted to request the user to change the desired minimal volume $V_{min}$ and/or the desired maximal volume $V_{max}$ so that the estimated volume $V_{est}$ is within the range defined by and including the minimal volume $V_{min}$ and the maximal volume $V_{max}$. This rather is considered as a last resort, when the other volume adaption means of steps S333 or S335 do not offer satisfactory alternatives or are not sufficient.

In step S333 depicted in Fig. 10 the tolerance range for the target body temperature may be adapted in order to reduce the volume to be infused. The larger the target temperature tolerance range, the less infusion fluid is required. As shown in Figs. 3 to 6, different first and second target temperature tolerance ranges $Ra_1$, $Ra_2$ may be defined for the plurality of phases. By varying these tolerance ranges, also the estimated volume $V_{est}$ may be changed. E.g. by narrowing the first and/or target temperature tolerance range $Ra_1$, $Ra_2$ the estimated volume $V_{est}$ increases and vice versa. On the other hand, a narrow first and/or target temperature tolerance range $Ra_1$, $Ra_2$ increases the control quality. As shown in Fig. 10, by widening the first and/or second tolerance range the estimated volume $V_{est}$ may be shifted to a value with the range defined by the minimal volume $V_{min}$ and the maximal volume $V_{max}$. The user may set maximal values for the first and second temperature tolerance range. The first minimal tolerance range may be limited by the machine configuration.

For instance, the first lower threshold may be less than 0,5°C, preferably less than 0,25°C, most preferably less than 0,15°C below target body temperature $T_{b\ tar}$. The first upper threshold $T_{u1}$ may be less than 0,5°C, preferably less than 0,25°C, most preferably less than 0,15°C above target body temperature $T_{b\ tar}$. The resulting first target body temperature tolerance range may be may be less than 1°C, preferably less than 0,5, most preferably less than 0,3°C. The first and second upper and lower thresholds may be varied for each of the plurality of phases C, H, W. Moreover, a third upper and/or third lower threshold may be defined, wherein the controller 100 is adapted to trigger an audible and/or visual alarm if the actual temperature reaches one of the third upper or lower threshold.

Also shown in Fig. 9 is the effect of an adapted treatment time on the estimated volume $v_{est}$. By lengthening the treatment time the estimated volume $V_{est}$ old being less than the minimal volume $V_{min}$ may be shifted to a new value $V_{est\ new}$ being within the range defined by minimal volume $V_{min}$ and maximal volume $V_{max}$. Similarly, by shortening the treatment time the estimated volume $V_{est\ old}$ may be shifted to a new or changed value $V_{est\ new}$ being lower than the previous estimated volume $V_{est\ old}$. Thus the shortened target treatment time $t_{treatment\ new}$ and/or the extended target treatment time $t_{treatment\ new}$ may be selected such that the changed estimated volume $V_{est\ new}$ is within the range defined by and including the minimal volume $V_{min}$ and the maximal volume $V_{max}$.

As depicted in Figs. 11 to 13 referring to step S335, the controller 100 may be adapted to optimize the volume to be infused. Preferably, the controller 100 is adapted to reduce the volume of infusion fluid to be infused either to reduce the infusion volume within the range defined by and including the minimal volume $V_{min}$ and the maximal volume $V_{max}$ (cf. Fig. 12) or so as to shift the estimated volume $V_{est\ old}$ from a value outside said range to a changed new value $V_{est\ new}$ being inside said range (cf. Fig. 11). It is assumed that the estimated volumes $V_{est\ new}$ and $V_{est\ old}$ both are above the minimal volume $V_{min}$.

The controller 100 may optimize the volume based on the consideration of the different control sensitivities of the at least two different temperature adaption means 200, 300. The control sensitivity is a measure of a change of the respective controller output value(s), here the body temperature, relative to a change of the controller input value(s) of the temperature adaption device 200, 300. The control sensitivity is a measure of the effectiveness of the adaption means. E.g. for the infusion device the sensitivity may be calculated with the formula:

$$(4) \quad S_{200} = DELTA\ Tb\ /\ DELTA\ V$$

wherein:

| | |
|---|---|
| $S_{200}$ | is the control sensitivity of the infusion device 200 |
| DELTA $T_{b\ act}$ | is the change of the actual body temperature, and |
| DELTA V | is the volume infused to achieve the change of actual body temperature, |

provided that infusion device 200 changes the body temperature by varying the volume flow of infused fluid. Preferably, the fluid temperature is constant for cooling (preferably about 4°C) and heating (preferably about 42°C). A high sensitivity means that a high change in body temperature is achieved with a given fluid volume and vice versa.

E.g. for a heat exchanging pad, the control sensitivity may be calculated with the formula:

$$(5) \quad S_{300} = DELTA\ Tb\ /\ DELTA\ T_{heat\ exchange}$$

wherein:

| | |
|---|---|
| $S_{300}$ | is the control sensitivity of the pad 300 |
| DELTA $T_{b\ act}$ | is the change of the actual body temperature, and |
| DELTA $T_{heat\ exchange}$ | is the surface temperature of the pad being in contact with the patient, |

provided that the body temperature is changed by varying the temperature of the heat exchange surface.

The control sensitivity of the infusion device may vary over time. For instance the control sensitivity changes if the temperature of the infusion fluid is varied during the treatment. For instance, the controlling sensitivity is different for heating and cooling by infusion device 200 since the temperature difference of the infused fluid to the blood temperature is higher for cooling, i.e. infusion of about 4°C cold infusion fluid, than for heating, i.e. infusion of about 42°C warm infusion fluid.

Different values for the control sensitivity may be defined for the different treatment phases C, H, W. Also different sensitivity values may be defined for heating and cooling, e.g. pre-set values for heating and for cooling with temperature adaption device 200. The control sensitivity S may be estimated based on user input, e.g. desired settings and/or machine core data, e.g. by using data of known temperature devices stored in the apparatus. The controller may be adapted to estimate at least one first estimated volume portion $V_{est\ 1}$ of fluid to be infused with a first control sensitivity $S_{200-1}$, preferably of the infusion device 200. The controller may be adapted to estimate at least one second estimated volume portion $V_{est\ 2}$ of fluid to be infused with a second control sensitivity $S_{200-2}$, for instance of the infusion device 200, wherein the first control sensitivity $S_{200-1}$ may be higher than the second control sensitivity $S_{200-2}$. The first control sensitivity $S_{200-1}$ may be at least 1,2 times, preferably at least 2 times, further preferred at least 4 times and most preferred at least 6 times higher than second control sensitivity $S_{200-2}$. E.g. the first control sensitivity $S_{200-1}$ may be achieved during cooling, thus, during a time period with high temperature difference between infusion fluid and blood. Moreover, the second control sensitivity $S_{200-2}$ may be achieved during heating, thus, during a time period with low temperature difference of infusion fluid and blood. The controller is not limited to a first and a second control sensitivity level and may also be configured to determine a plurality of volume portions, for a plurality of control sensitivity levels. Moreover, the first estimated volume portion $V_{est\ 1}$ and the second estimated Volume portion ($V_{est\ 2}$) are not limited to portions in one consecutive time period. The estimated volume portion at a particular sensitivity level may be the sum of all volumes infused with the same sensitivity level during the treatment. The estimated volume $V_{est}$ may be the sum of the first estimated volume portion $V_{est\ 1}$ and the second estimated volume portion $V_{est\ 2}$.

The underlying idea of the volume optimisation under consideration of the control sensitivity is that the controller operates the infusion device 200 preferably if infusion is considered to be highly effective, i.e. high control sensitivity $S_{200-1}$. In time periods where the infusion device 200 is not considered to be highly effective, i.e. low control sensitivity $S_{200-2}$, the controller predominantly uses the further volume neutral temperature adaption means 300 if the further temperature adaption means 300 is suitable to achieve the target body temperature. In other words, the infusion with infusion device 200 at higher control sensitivities is prioritised over infusion with infusion device 200 at lower control sensitivities if infusion volume should be reduced. Moreover, if the infusion device 200 would operate at low control sensitivities the operation of the further temperature adaption means 300 may be prioritized.

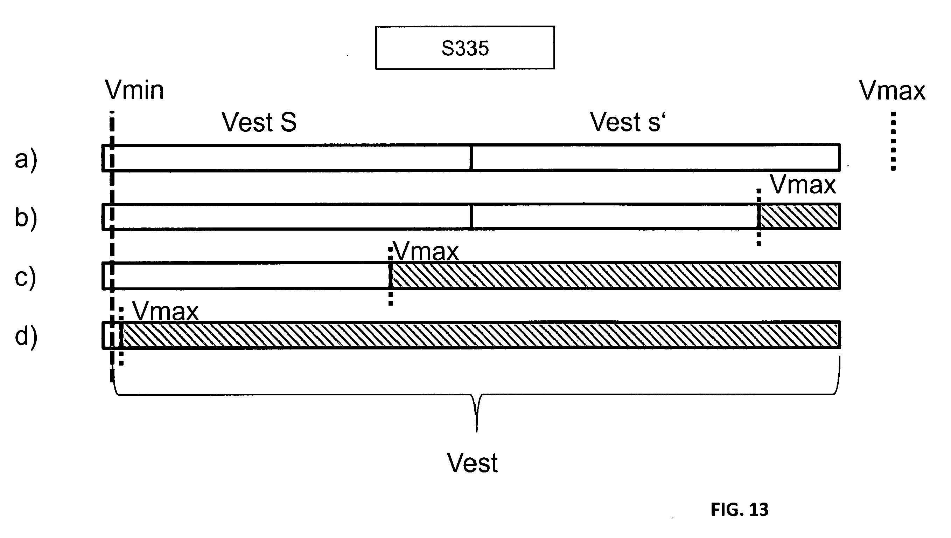

The control is now described referring to Fig. 13. In scenario a), the estimated volume $V_{est}$ for the desired target body temperature profile is within the range defined by and including $V_{min}$ and $V_{max}$. $V_{est\ S}$ is the volume which may be infused with a high sensitivity (i.e. for cooling) and $V_{est\ s'}$ is the volume required for infusion with a low sensitivity in order to achieve the desired target body temperature profile. The estimated volume $V_{est}$ is the sum of $V_{est}$ s and $V_{est\ s'}$. Since the estimated volume $V_{est}$ is within the range defined by $V_{min}$ and ($V_{max}$ a further optimisation of the volume is not required. However, if the clinical staff would like to further reduce the volume, the volume may be optimized. For instance the volume may be reduced by widening the tolerance range and/or the further volume neutral temperature adaption device may be prioritizes, and thus predominantly used. Also, the target values may be changed.

In scenario b) the estimated volume is not within the range defined by and including $V_{min}$ and $V_{max}$ but at least above the estimated volume $V_{est\ S}$ of scenario a), which may be infused with a high sensitivity. One possibility to shift the estimated volume $V_{est}$ may be to operate heating periods (i.e. periods of low control sensitivity of the infusion device), at least partially, only the further volume neutral temperature adaption device 300, e.g. an intravascular catheter 300. The time period, during which only the device 300 is operated, results in a savings in infusion fluid. Therefore, the length of the time period may be selected such that the estimated volume required for the target body temperature profile is within the range defined by and including $V_{min}$ and $V_{max}$. The areas of increased or high control sensitivity will be used completely and the use of the volume with low control sensitivity is reduced. In other words devices 200, 300 are operated and/or prioritized under consideration of the minimal volume $V_{min}$ and the maximal volume $V_{max}$.

In scenario c) the estimated volume $V_{est}$ is not within the range defined by and including the minimal volume $V_{min}$ and the estimated high control sensitivity volume $V_{est\ S}$ of scenario a). During time periods of relatively low control sensitivity, i.e. heating of the patient, no infusion fluid will be infused and only the volume neutral temperature adaption means will operate. Here, also during time periods of relatively low control sensitivity, i.e. cooling of the patient, the infusion of fluid may be reduced and the further volume neutral adaption means will also, preferably at least partially, operate.

In scenario d) the maximal volume ($V_{max}$ is almost equal to the minimal volume $V_{min}$. Consequently almost no volume may be used for the temperature adaption. The temperature adaption is predominantly to be achieved with the further temperature adaption means 300. For all scenarios a) to d) the controller is adapted to estimate whether the target body temperature profile $T_{b\ tar\ prof}$ may be achieved also when the infusion means at least partially is not operating.

While the prioritisation is described in Fig. 13 for the entire treatment, the prioritisation or volume optimisation may also be applied to the plurality of treatment phases C, H, W. The evaluation of scenarios a) to d) may be conducted during initial setup or during treatment. Moreover, the controller may be adapted to change the prioritization of the two different temperature adaption means 200, 300 automatically, preferably as long as the defined target temperature profile may be achieved.

Alternatively, the prioritisation or distribution of the volume to be infused may be described by the formula:

$$(6) \qquad V_{est} = a * V_{S\ est} + b * V_{s'\ est}$$

wherein:

$V_{est}$     is the total volume to be infused for the defined target body temperature profile,
$V_{S\ est}$     is the total volume to be infused for the defined target body temperature profile with a high sensitivity,
$V_{s'\ est}$     is the total volume to be infused for the defined target body temperature profile with a low sensitivity, and
a, b     are multipliers with values between 0 and 1.

Moreover within the plurality of treatment phases as well as the treatment phases it selves could be prioritizes, for instance according to the formula:

$$(7) \qquad V_{est} = a * V_{est\ C} + b * V_{est\ H} + c * V_{est\ W}$$

wherein:

$V_{est}$     is the total volume to be infused for the defined target body temperature profile,
$V_{est\ C}$, $V_{est\ H}$, $V_{est\ W}$     are the total volumes to be infused in order to achieve the defined target body temperature profile, wherein $V_{est\ C}$, $V_{est\ H}$, $V_{est\ W}$ may be calculated using formula (6), i.e. prioritization within respective treatment phase(s)
a, b, c     are multipliers with values between 0 and 1.

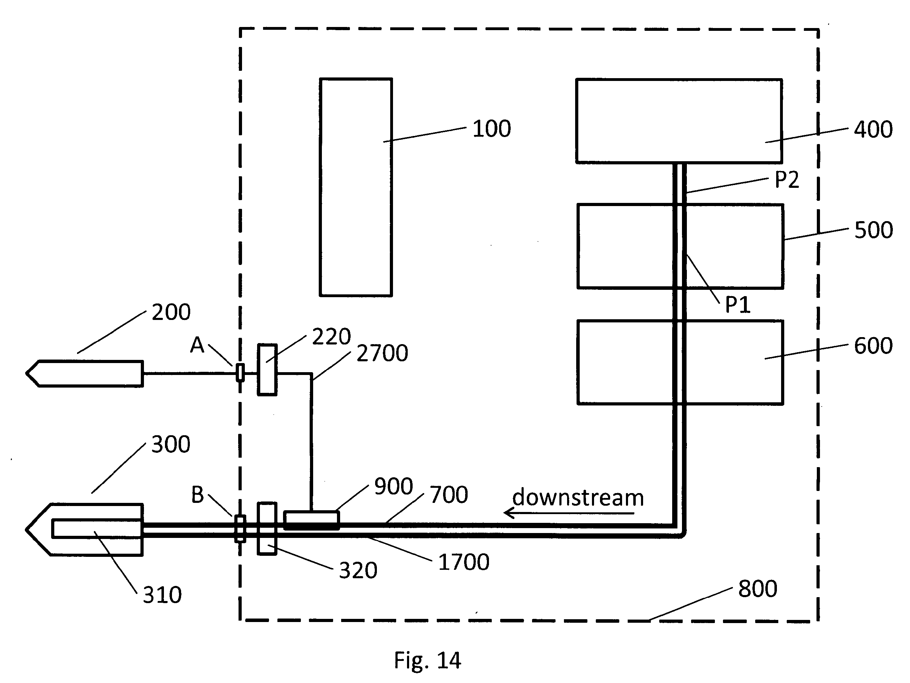

Fig. 14 depicts an apparatus for adapting a body temperature $T_b$. Here, the apparatus is connected to an infusion device 200, here configured as an infusion needle being connected via a tubing to a connector A of the apparatus. Herein after, the further temperature adaption device (300) is preferably an intravascular catheter. However the same applies for a pad comprising the heat exchanger (310). Catheter 300 comprising heat exchanger 310 is also connected via a second tubing to a connector B of the apparatus. The apparatus comprises here a fluid supply 400, a fluid temperature adaption device 500, here configured as a heat exchanger, and a fluid pump 600.

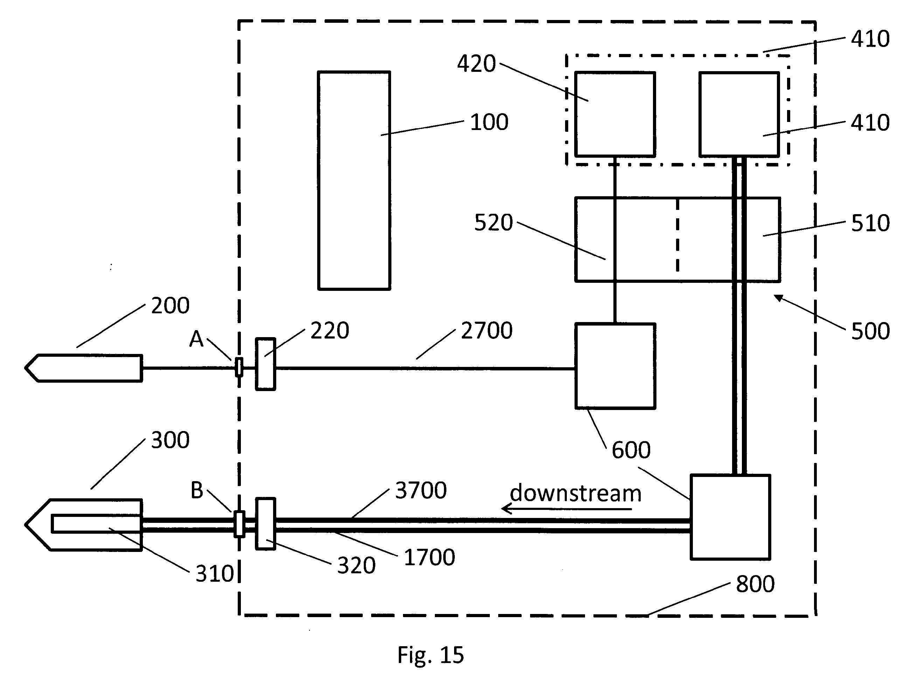

Fluid supply 400, heat exchanger 500 and fluid pump 600 may be fluidly interconnected by one fluid circuit. The fluid circuit may comprise a fluid portion 700 extending from the fluid supply 400 downstream to the heat exchanger 310 and a second fluid circuit portion 1700 extending from the heat exchanger 310 downstream back to fluid supply 400. In Fig. 14 and 15, the arrow indicates the flow direction away from the exit of fluid supply 400 to be the direction "downstream". An actuating device 900, here configured as a switch or valve or vent, may separate the incoming flow of fluid into two separate fluid flows. A first flow 2700 flows from the switch 900 towards the infusion needle 200. A second flow is directed from the device 900 to the heat exchanger 310. The measuring devices 220, 320 located downstream the valve 900 may measure the temperature or pressure or flow rate of the fluid flowing to the infusion needle 200 and to the catheter 300, respectively. Here, the infusion needle 200 and the intravascular catheter 300 share a fluid circuit portion 700 located upstream of the heat exchanger 311, more precisely upstream of valve 900.

The term sharing indicates that the shared component(s) is/are structurally and/or functionally interrelated to both the infusion device 200 and the intravascular catheter 300. In other words, the infusion device 200 and the intravascular catheter 300 are used or operated by or while using shared components. This means that the apparatus is adapted to operate the infusion device 200 and the intravascular catheter 300 by sharing at least one or more of the components: fluid circuit portion 700, controller 100, fluid supply 400, fluid temperature adaption device 500, pump 600, and/or housing 800 for operating the infusion device 200 and the intravascular catheter 300. Sharing the fluid circuit portions 700 means that both the infusion needle and the catheter 300 are provided with fluid flowing at least in one section through the same fluid path.

In Fig. 14, infusion needle 200 and catheter 300 are configured as separate devices. Alternatively, infusion needle 200 may be integrated in catheter 300. For instance, infusion needle 200 may be located adjacent to the heat exchanger 310, preferably such that both devices are inserted into the patient at the same time using the same opening.

More preferably, infusion needle 200 and catheter 300 are connected to the apparatus with a shared tubing comprising a flow path to the infusion needle and a further flow path from the connector to the heat exchanger 310 and back. Both flow paths are separated from each other, and preferably thermally isolated. The isolation may be configured as an increased material layer of a material with low heat conductivity such as a resin material. The increase material layer may exceed the wall thickness of the flow channels. The layer may be located between the flow channels. If a shared tubing is used then the fluid coming back from the heat exchanger may negatively affect the temperature of the fluid to be infused flowing through a flow channel located directly next to the flow flowing back from the heat exchanger 310. The thermal isolation may reduce the impact of the relatively warm fluid flow on the fluid to be infused.

Furthermore the shared tubing may comprise isolation means provided between the individual flow channels, particularly to provide isolation between the flow path of the infusion needle and the flow path of the additional temperature adjusting device. This may be of advantage in case of targeted opposite temperature profiles of the two different devices. The targeted temperature adjustment of the patient's body core temperature via the infusion needle 200 may be a fast systemic cooling of the patient via rapid inflow of cold saline, this being beneficial from the medical view, however rather uncomfortable for the patient. Consequently the second temperature adjusting device may improve patient's comfort via, e.g., surface pads on the skin that are tempered above the normal body temperature to increase patients comfort, while systemically cooling the body via the rapid infusion of cold fluid, e.g., saline. This abovementioned isolation means may only be required along a portion of the shared tubing.

The shared tubing may be connected to the apparatus via a shared connector being adapted to mate with a shared connector of the apparatus. The term "shared connector" means that the flow path to the catheter 300 (and back) as well as the flow path to the infusion needle 200 are fluidly connected to the corresponding flow paths of the apparatus, preferably by a single coupling or connecting operation. The volume of the infused fluid is preferably controlled by a controller 100 as explained above. Not depicted in Fig. 14 are the connections of controller 100 with the different components of the apparatus. For instance, controller 100 may be operatively coupled to the heat exchanger 500, the pump 600, the valve 900 and/or the measuring means 220, 320. Dashed line 800 of Fig. 14 depicts the housing of the apparatus. While all components are here located in one housing 800, also different modular concepts with different housings as discussed herein with reference to Fig. 1 may be applied.

Compared to prior art devices, the apparatus depicted in Fig. 14 comprises less components leading to reduced manufacturing costs. Moreover, the required space may be reduced. Furthermore, since only one apparatus instead of two apparatus needs to be set up, also the set up time may be shortened. The shared tubing and the shared connector may further reduce the costs and set up time. Moreover, the shared tubing may also improve the accessibility of the patient since the additional tubing for the infusion is eliminated and will not disturb the clinical staff anymore. If the infusion needle is integrated into the catheter, the set up time may be further reduced and the risk of infections may also be further decreased. On the other hand, having two different devices may ensure that still a cooling is induced while the other device may be replaced.

Fig. 15 shows an apparatus with a different fluid temperature adaption device 500. The fluid temperature adaption device 500 is here configured as a heat exchanger comprising two different flow paths for the fluid flows 2700 and 3700. Fluid flow 2700 interconnects infusion needle 200 with a supply of infusion fluid 420. Flow path 3700 interconnects the exit of the fluid supply 410 for the heat exchanger with the heat exchanger 310 of the catheter 300. Fluid flows 2700, 3700 may be separated from each other. Having separated fluid flows ensures that the fluid used in the catheter 300 may not be mixed with the fluid flow 2700 to be infused into the body of a patient. Consequently, the risk of a contamination of the fluid to be infused into the body of the patient may be further reduced. The different flow paths of fluid flows 2700, 3700 may be spaced apart from each other. Heat exchanger 500 may comprise a first section 520 for the temperature adaption of the fluid to be infused to the patient and a second section 510 in which the temperature of the fluid flowing to the heat exchanger 310 of the catheter is adapted. Depicted in Fig. 15 are also two pumps 600 adapting the flow rate of the two separate fluid flows 2700, 3700. The separate fluid supplies 410, 420 may be located in a container 430 being thermally isolated.

The systems for adapting a body temperature depicted in Figs. 14 and 15 may allow reducing the at least one of: manufacturing costs, set up time, cabling, and risk of infection. In addition, the ease of use and patient comfort may be further increased. By using the controller adapted to control the infusion means 200 and the at least further body temperature adaption means 300, here preferably configured as a catheter 300, it is advantageously also possible to further improve the control accuracy of the body temperature treatment and/or to adjust the volume to be infused to the patient to a desired level.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality.

**Claims**

1. Apparatus for adapting a body temperature ($T_b$), connected or connectable to an infusion device (200), preferably configured as an infusion needle, and a further temperature adaption device (300) comprising a heatexchanger (310), preferably configured as an intravascular catheter or pad comprising the heatexchanger (310), wherein during use the infusion device (200) and the further temperature adaption device (300) share:

   - a fluid circuit portion (700) through which fluid infusible to the patient and fluid intended for heat exchange in the further temperature adaption device (300) flow;
   - a controller (100) adapted to control the body temperature ($T_b$) by controlling the infusion of the infusion device (200) and the heat exchange of the further temperature adaption device (300); and/or
   - a fluid supply (400) providing fluid for adapting the body temperature.

2. The apparatus according to claim 1, wherein the infusion device (200) and the further temperature adaption device (300) share:

   - a fluid temperature adaption device (500) adapted to change both the temperature of the fluid infusible to the patient and the fluid intended for heat exchange in the further temperature adaption device (300);
   - a pump (600) adapted to pump at least one of fluid infusible to the patient and fluid intended for heat exchange in the further temperature adaption device (300); and/or
   - a housing (800).

3. The apparatus of any one of the preceding claims, wherein the infusion device (200) is adapted to add a volume of fluid to the patient's blood circuit, the volume of fluid having a different temperature than the body temperature ($T_b$).

4. The apparatus according to any one of the preceding claims, wherein the fluid infusible to the patient and the fluid intended for heat exchange in the further temperature adaption device (300) are the same biocompatible fluid, preferably NaCl or other balanced fluids like ringer's solution.

5. The apparatus according to any one of the preceding claims, wherein the infusion device (200) and the intravascular catheter (300) in fluid connection with the apparatus are adapted to be applied to blood vessels at different locations.

6. The apparatus according to any one of the preceding claims, wherein the intravascular catheter (300) is applied into the central venous system, e.g. via femoral, subclavian or internal jugular insertion, and wherein the infusion device (200) is applied at a peripheral location, e.g. a venous access at the crook of the arm.

7. The apparatus according to any one of the preceding claims, wherein the apparatus (100) is adapted to selectively and/or simultaneously operate the infusion with the infusion device (200) and the heat exchange with the further temperature adaption device (300).

8. The apparatus of any one of the preceding claims, wherein the controller (100) is adapted to control the body temperature ($T_b$) by simultaneously controlling the infusion with the infusion device (200) and the heat exchange with the further temperature adaption device (300).

9. The apparatus of any one of the preceding claims, further comprising at least one measuring device (220, 320) adapted to measure the pressure and/or the temperature of the fluid.

10. The apparatus of claim 79, further comprising two measuring devices (220, 320), one (220) measuring the pressure and/or the temperature of the fluid infusible to the patient and the other (320) measuring the pressure and/or the temperature of the fluid intended for heat exchange in the intravascular catheter.

11. The apparatus of any one of the preceding claims, further comprising a shared connector, wherein the shared connector is adapted to mate with a corresponding connector shared by the infusion means (200) and the further temperature adaption device (300), and/or wherein the temperature adaption device (500) is a heat exchanger and/or wherein the fluid temperature may be changed by mixing two fluids of different temperatures.

12. The apparatus according to any one of the preceding claims I to 11, fluidly connected or connectable to an infusion device (200) and an further temperature adaption device (300), wherein the infusion device (200) and the further temperature adaption device (300) share a fluid circuit portion (700), the fluid circuit portion (700) at least being located

   - downstream of a fluid temperature adaption device (500) adapted to change both the temperature of the fluid infusible to the patient and the fluid intended for heat exchange in the further temperature adaption device (300), and
   - upstream of a heat exchanger (310) located in the further temperature adaption device (300).

13. The apparatus according to any one of the preceding claims, connected or connectable to an infusion device (200) and a further temperature adaption device (300), wherein the infusion device (200) and the further temperature adaption device (300) share a fluid temperature adaption device (500), wherein a flow of fluid infusible to the patient and a flow of fluid intended for heat exchange in the further temperature adaption device (300) are two separate fluid flows (2700, 3700) flowing through the fluid temperature adaption device (500).

14. System for adapting a body temperature ($T_b$) comprising:

   - the apparatus in accordance with any one of the preceding claims 1 to 13;
   - an infusion device (200); and
   - an intravascular catheter (300).

15. The system of claim 14, wherein the infusion device (200) is located in or at the intravascular catheter (300), preferably so that a heat exchange portion (310) of the catheter (300) and the infusion device (200) are jointly insertable into the patient through the same opening.

FIG. 1

FIG. 2

EP 2 932 946 A1

FIG. 3

FIG. 4

FIG. 5

FIG. 6

EP 2 932 946 A1

```
┌─────────────────────────────────────────┐
│      S10: input target parameters        │◀──────────┐
└─────────────────────────────────────────┘           │
                    │                                   │
                    ▼                                   │
┌─────────────────────────────────────────────┐        │
│         S20: plausabilty check              │        │
└─────────────────────────────────────────────┘        │
      ok │                    not ok                    │
         ▼                       ◇ change target parameters?
┌──────────────────────┐        ╱◇╲                      │
│   S30: optimisation  │◀──     ◇   ◇  ─────── yes ──────┘
└──────────────────────┘         ◇ 
         │                 │ no
         │          yes    ▼
         │              ╱◇◇╲ continue treatment?
         │              ◇   ◇
         ▼               ◇
┌──────────────────────────────┐    no
│ S40 start/continuation of    │      ▼
│       treatment              │   ┌────────────────────┐
└──────────────────────────────┘   │ S70: stop treatment│
         │                         └────────────────────┘
         ▼
┌──────────────────────────────────────────┐
│ S50: actualisation of estimated values;   │
│ verification: target parameter can be      │
│                achieved?                   │
└──────────────────────────────────────────┘
    yes │                      no
        ▼
┌──────────────────────────────┐
│   S60:  continue treatment   │
└──────────────────────────────┘

   ◇  decision by user
```

**FIG. 7**

FIG. 8

S30: optimisation

S31: calculation Vest
suggest target body temperature profile

target body temperature profile acceptable?

yes

no

S321: Vest < Vmin

S322: Vmin < Vest < Vmax

S323: Vest > Vmax

S331: add volume?

S332: adapt Vmin and/or Vmax?

S333: adapt tolerance range?

S334: adapt treatment period?

S335: Volume optimisation

S34: calculate Vest new
Vmin < Vest new < Vmax?

no

yes

S40

Vmin | S331 | S333 | S334 | Vmax

**FIG. 9**

Vest old          Vest new

Vmin new   Vmin old   | S332 |   Vmax

**FIG. 10**

Vest

Vmin | S333 | S334 | S335 | Vmax

**FIG. 11**

Vest new   Vest old

Vmin | S335 | Vmax

**FIG. 12**

Vest new          Vest old

FIG. 13

Fig. 14

Fig. 15

Europäisches
Patentamt

European
Patent Office

Office européen
des brevets

# EUROPEAN SEARCH REPORT

Application Number

EP 14 00 1426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/235496 A1 (COLLINS KENNETH A [US] ET AL) 19 October 2006 (2006-10-19) | 1-14 | INV.<br>A61F7/12<br>A61M5/44 |
| Y | * paragraphs [0031], [0032], [0034], [0035], [0036], [0038], [0041], [0047]; figure 3 * | 15 | |
| A,D | EP 2 698 182 A1 (SEIRATHERM GMBH [DE]) 19 February 2014 (2014-02-19) * paragraph [0028] * | 9,10 | |
| Y | US 6 719 724 B1 (WALKER BLAIR D [US] ET AL) 13 April 2004 (2004-04-13) * abstract * | 15 | |
| A | WO 2012/143479 A1 (SEIRATHERM GMBH [DE]; ROTH MATTHIAS [DE]; BAENKLER MARC [DE]) 26 October 2012 (2012-10-26) * abstract * | 1-15 | |

**TECHNICAL FIELDS SEARCHED (IPC)**

A61F
A61M

The present search report has been drawn up for all claims

| Place of search | Date of completion of the search | Examiner |
|---|---|---|
| The Hague | 25 September 2014 | Mayer-Martenson, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 00 1426

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2014

| Patent document cited in search report | | Publication date | Patent family member(s) | | Publication date |
|---|---|---|---|---|---|
| US 2006235496 | A1 | 19-10-2006 | US | 2006235496 A1 | 19-10-2006 |
| | | | US | 2011137249 A1 | 09-06-2011 |
| EP 2698182 | A1 | 19-02-2014 | EP | 2698182 A1 | 19-02-2014 |
| | | | WO | 2014026977 A1 | 20-02-2014 |
| US 6719724 | B1 | 13-04-2004 | NONE | | |
| WO 2012143479 | A1 | 26-10-2012 | CN | 103596609 A | 19-02-2014 |
| | | | EP | 2514453 A1 | 24-10-2012 |
| | | | US | 2014148881 A1 | 29-05-2014 |
| | | | WO | 2012143479 A1 | 26-10-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2203136 A1
- WO 2006091284 A1
- WO 02098483 A1
- EP 2514453 A
- WO 2009056640 A3
- EP 2698182 A

**Non-patent literature cited in the description**

- **KOLLMAR et al.** Therapeutische Hypothermie - Prinzip, Indikationen, praktische Anwendung. Unimed-Verlag, 2011